# EUROPEAN PATENT SPECIFICATION

(11) **EP 0 870 828 B1**
(45) Date of publication and mention of the grant of the patent: **30.11.2005**
(21) Application number: 97940361.5
(22) Date of filing: 12.09.1997
(51) Int. Cl.: C12N 15/31, C12P 21/02, C12P 21/08, C12Q 1/68, G01N 33/53, C12N 1/21, C07K 14/195, C07K 16/12, A61K 39/42

(54) **NOVEL POLYPEPTIDE ORIGINATING FROM HEMOPHILUS PARAGALLINARUM AND PROCESS FOR PRODUCING THE SAME**
AUS HEMOPHILUS PARAGALLINARUM ABSTAMMENDES POLYPEPTID UND VERFAHREN ZU DESSEN HERSTELLUNG
NOUVEAU POLYPEPTIDE EXTRAIT DE HEMOPHILUS PARAGALLINARUM ET SON PROCEDE DE PRODUCTION

(30) Priority: 19.09.1996 JP 27140896
(43) Date of publication of application: 14.10.1998
(73) Proprietor: Juridical Foundation, The Chemo-Sero-Therapeutic Research Institute, Kumamoto-shi, Kumamoto 860-8568 (JP)
(72) Inventor: TOKUNAGA, Eiji, Kumamoto-shi Kumamoto 860 (JP); SAKAGUCHI, Masashi, Kumamoto-shi Kumamoto 860 (JP); MATSUO, Kazuo, Kumamoto-shi Kumamoto 860 (JP); HAMADA, Fukusaburo 2679-2, Suya, Kumamoto 861-11 (JP); TOKIYOSHI, Sachio, Kumamoto-shi Kumamoto 862 (JP)
(74) Representative: Vossius & Partner
(86) International application number: PCT/JP1997/003222
(87) International publication number: WO 1998/012331

(56) References cited:
- JP-A- 1 000 467
- JP-A- 5 112 466
- JP-A- 8 027 028
- JP-A- 56 045 416
- JP-A- 56 115 724
- DATABASE SRS [Online] 9 August 1995 (1995-08-09) FLEISCHMANN RD ET AL.: "Haemophilus influenzae genome" Database accession no. U32845 XP002200265
- BEN-YEHUDA A ET AL: "Recombinant vaccinia virus with influenza hemagglutinin protects old mice from influenza infection." TRANSACTIONS OF THE ASSOCIATION OF AMERICAN PHYSICIANS. UNITED STATES 1992, vol. 105, 1992, pages 177-181, XP002200264 ISSN: 0066-9458
- OHUCHI M ET AL: "MUTATIONS AT THE CLEAVAGE SITE OF THE HEMAGGLUTININ ALTER THE PATHOGENICITY OF INFLUENZA VIRUS A/CHICK/PENN/83 (H5N2)" VIROLOGY, ACADEMIC PRESS,ORLANDO, US, vol. 2, no. 168, 1989, pages 274-280, XP001068975 ISSN: 0042-6822
- JACOBS A A C ET AL: "EFFICACY OF A TRIVALENT HAEMOPHILUS-PARAGALLINARUM VACCINE COMPARED TO BIVALENT VACCINES" VETERINARY MICROBIOLOGY, vol. 32, no. 1, 1992, pages 43-49, XP001070372 ISSN: 0378-1135
- VET. MICROBIOL., 34, (1993), M. TAKAGI et al., "Purification of Hemaglutinin from Haemophilus Paragallinarum Using Monoclonal Antibody", pages 191-197.

## Description

### TECHNICAL FIELD

The present invention relates to a polypeptide which can prevent avian infectious coryza. More particularly, the present invention relates to a polypeptide from Haemophilus paragallinarum, the causative agent of avian infectious coryza, a gene coding for said polypeptide and an antibody protein which recognizes said polypeptide. The present invention further relates to a process for preparing said polypeptide and the use of said polypeptide for a vaccine, a diagnostic agent and a therapeutic agent.

### BACKGROUND ART

Avian infectious coryza is one of the most important respiratory diseases in poultry, which is an acute respiratory disease caused by infection with Haemophilus paragallinarum (hereinafter also referred to as "HPG") with cardinal symptoms being a running nose, swelling of the face and epiphora. Avian infectious coryza brings about a great economical damage since it leads to decrease in the breeding rate of poultry, retarding of egg laying, decrease in egg production or failure of egg laying. For prevention of avian infectious coryza, an inactivated vaccine has hitherto been used widely which is obtained by culturing Haemophilus paragalinarum, recovering and inactivating the cells with formalin, thimerosal and the like. However, adverse side effects caused by such an inactivated vaccine has been an issue as it has been reported that local necrotic lesions are formed in the inoculated chicken when the vaccine is administered (M. Matsumoto and R. Yamamoto, Avian Dis., 15: 109-117, 1971), and hence, development of a highly safe vaccine is earnestly desired.

In recent years, laborsaving in breeding and managing poultry is in progress with a scale-up of breeding poultry. As a part of this, laborsaving in vaccination has also been earnestly desired, and as a result, a mixed vaccine has already been developed and widely used in the field so that a frequency of inoculation can be reduced by mixing several kinds of vaccines together.

In order to provide a mixed vaccine showing immunogenicity equivalent to that of each plain vaccine without increase of dosage amount, it is necessary to increase an amount of each antigen contained in a mixed vaccine or to find out and use a more suitable adjuvant. However, in case of gram-negative bacteria such as HPG, a higher amount of antigen is likely to enhance a response to injection such as swelling at the inoculated site. Therefore, in order to reduce such an adverse response, it is preferable to obtain only a protective antigen, i.e. an effective component, from bacterial cells or culture supernatant, or to clone a gene coding for said antigen by the genetic recombination technique, to express said gene in bacteria, yeast, an animal cell, a plant cell, an insect cell and the like, and to purify a product expressed in a large amount, which is then mixed with an appropriate adjuvant together with other vaccines.

Another approach for laborsaving of vaccination is the use of virus or bacteria as a vector. That is, genes coding for protective antigens from one or plural pathogens have been incorporated into an attenuated virus or bacteria to prepare a polyvalent live vaccine. For fowls, poxvirus, Marek's disease virus and the like have been investigated as a vector. A vaccine comprising a viral vector has been put into practice wherein genes coding for HN and F proteins of Newcastle disease virus are incorporated into fowl pox virus.

It is thus most important to identify a protective antigen of HPG for development of a safe and effective vaccine against avian infectious coryza both as a component vaccine and as a vector vaccine.

Among protective antigens of HPG such as hemagglutinin (HA) and outer-membrane protein, HA is considered a most important antigen since immunization of chicken with HPG increases a hemagglutination-inhibition antibody (hereinafter referred to as "HI antibody") and higher protective effect is observed for chickens with high level of HI antibody (K. Otsuki and Y. Iritani, Avian Dis., 18: 297-304, 1974 and K. Kume et al., Jpn. J. Vet. Sci., 46: 843-850, 1984).

Serotype of HPG is classified into serotypes A, B and C (Page, Am. J. Vet. Res., 23: 85-95, 1962) or into serotypes 1 and 2 (Sawata et al., Jpn. J. Vet. Sci., 40: 645-652, 1978) based on the agglutination test. It is considered that serotype A by Page corresponds to serotype 1 by Sawata et al. whereas serotype C by Page corresponds to serotype 2 by Sawata et al. (K.Kume, et al., Am. J. Vet. Res., 41: 757-760, 1980 and Sawata et al., Am. J. Vet. Res., 41: 1901-1904, 1980).

Kume et al. reported that HPG serotype A (serotype 1) has at least three kinds of HA, i.e. HA-L (heat-labile, trypsin-sensitive), HA-HL (heat-labile, trypsin-resistant) and HA-HS (heast-stable, trypsin-resistant), and that HA-L alone exhibits not only HA activity to usual fresh chicken erythrocytes but also to glutaraldehyde-fixed chicken erythrocytes and is involved in protection against infection with HPG serotype A (K. Kume, Jpn. J. Vet. Sci., 45: 783-792, 1983 and Sawata et al., Jpn. J. Vet. Sci., 46: 21-29, 1984).

Iritani et al. reported that HPG serotype A has two kinds of HA, i.e. type 1 HA (heat-labile, protease-sensitive) and type 2 HA (heat-labile, protease-resistant), and that type 1 HA, which is heat-labile and protease-sensitive and consisted of a polypeptide having a molecular weight of about 39 kd as a subunit, is involved in protection against infection (T. Yamaguchi and Y Iritani, Jpn. J. Vet. Sci., 42: 709-711, 1980 and Y. Iritani et al., Am. J. Vet. Res., 41: 2114-2118, 1980). It is considered that HA-L and HA-HL by Kume et al. correspond to type 1 HA and type 2 HA by Iritani et al., respectively. As to HPG serotype C (serotype 2), Sawata et al. reported that an antigen was found which is heat-labile and trypsin-sensitive and exhibits the HA activity to glutaraldehyde-fixed chicken erythrocytes and that this antigen is distinct from HA of HPG serotype A in their antigenicity (Sawata et al., Am. J. Vet. Res., 43: 1311-1314, 1982). However, to date, a protective antigen of HPG has not yet materially identified except for type 1 HA produced by HPG serotype A as reported by Iritani et al.

As mentioned hereinabove, the conventional inactivated vaccine obtained by inactivating Haemophilus paragallinarum cells with thimerosal, formalin and the like has provoked problems that the adverse side effects as mentioned above are induced when it is applied to fowls at a large amount since it includes various substances from the cells other than the protective antigen.

### DISCLOSURE OF INVENTION

The inventor has earnestly studied in order to solve the problems, and as a result, has successfully purified, from a culture supernatant of Haemophilus paragallinarum serotype A, a polypeptide having about 130 kd of molecular weight from Haemophilus paragallinarum serotype A, said polypeptide inducing production of HI antibody and protecting against avian infectious coryza by Haemophilus paragallinarum serotype A.

Furthermore, the present inventor has prepared a genomic DNA library from HPG serotype A, cloned a gene fragment coding for the above 130 Kd polypeptide expressed said gene fragment in E. coli and has found that the produced polypeptide could prevent avian infectious coryza by Haemophilus paragallinarum serotype A. Said gene fragment coding for the above 130 Kd polypeptide was also used as a probe for cloning a gene fragment hybridizable with said DNA fragment from HPG serotype C to give E. coli which expresses the polypeptide from HPG serotype C.

The present invention provides a safer, effective vaccine against avian infectious coryza, pathogenic bacteria of which is Haemophilus paragallinarum, with less adverse side effects and a process for preparing the same.

That is, an object of the present invention is to provide a novel polypeptide from Haemophilus paragallinarum as well as a peptide which shares at least a portion of the amino acid sequence.

Another object of the present invention is to provide a gene coding for said novel polypeptide from Haemophilus paragallinarum as well as the peptide which shares at least a potion of the amino acid sequence and a recombinant vector for expression of said gene.

Still another object of the present invention is to provide a process for preparing said novel polypeptide from Haemophilus paragallinarum and the polypeptide which shares at least a portion of the amino acid sequence from microorganisms or cells transformed with said recombinant vector.

Still further object of the present invention is to provide a monoclonal or polyclonal antibody which is prepared by using as an immunogen the thus prepared novel peptide from Haemophilus paragallinarum or the polypeptide which shares at least a portion of the amino acid sequence.

Still another object of the present invention is to provide a method for detecting Haemophilus paragallinarum or an antibody thereto by a combination of the above-mentioned peptide, DNA fragment, transformant or antibody.

Still further object of the present invention is to provide a therapeutic agent for avian infectious coryza which comprises as an active ingredient the antibody against the novel polypeptide from Haemophilus paragallinarum.

### BRIEF DESCRIPTION OF DRAWINGS

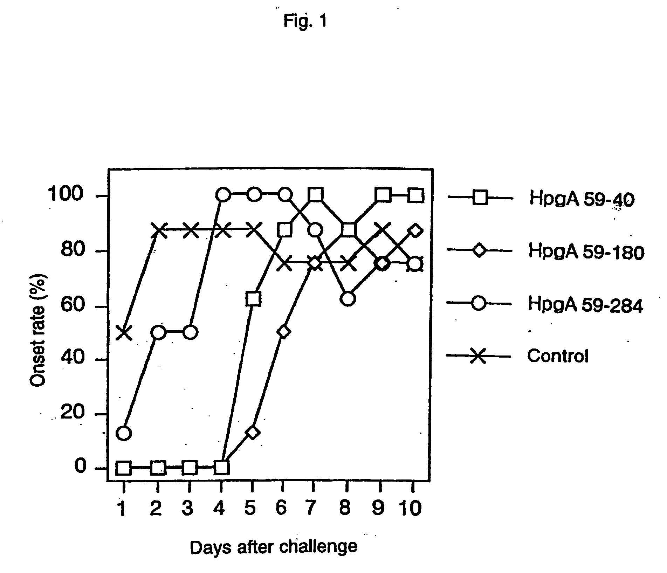

Figure 1 shows the results obtained by challenging chickens with Haemophilus paragallinarum serotype A strain 221 after passive immunization with monoclonal antibodies (clones HpgA 59-40, HpgA 59-180 and HpgA 59-284) wherein the onset of the disease was retarded in the groups previously administered with the monoclonal antibodies having the HI activity (clones HpgA 59-40 and HpgA 59-180).

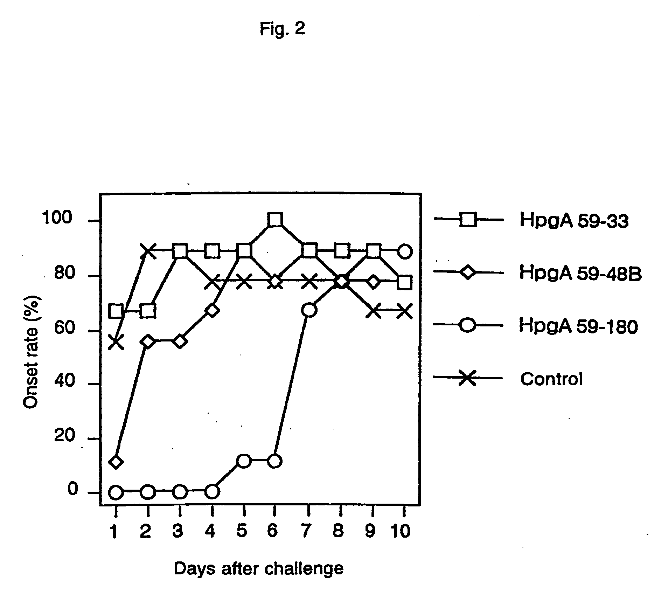

Figure 2 shows the results obtained by challenging chickens with Haemophilus Paragallinarum serotype A strain 221 after passive immunization with monoclonal antibodies (clones HpgA 59-33, HpgA 59-48B and HpgA 59-180) wherein the onset of the disease was retarded in the groups previously administered with the monoclonal antibody having the HI activity (clone HpgA 59-180).

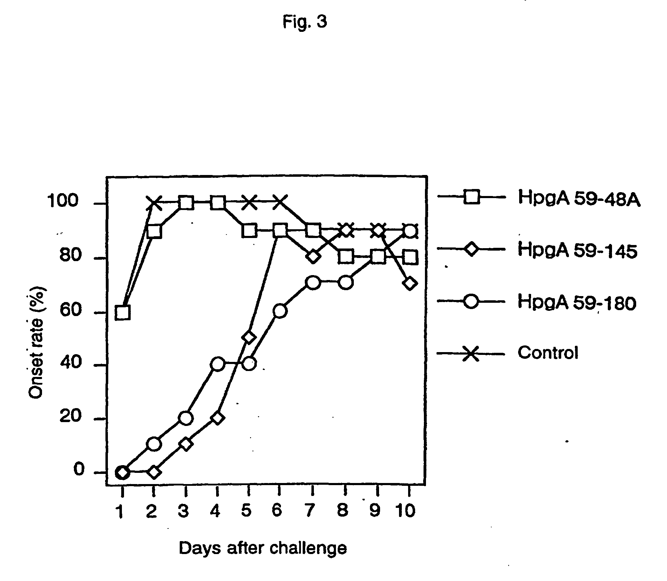

Figure 3 shows the results obtained by challenging chickens with Haemophilus paragallinarum serotype A strain 221 after passive immunization with monoclonal antibodies (clones HpgA 59-48A, HpgA 59-145 and HpgA 59-180) wherein the onset of the disease was retarded in the groups previously administered with the monoclonal antibodies having the HI activity (clones HpgA 59-145 and HpgA 59-180).

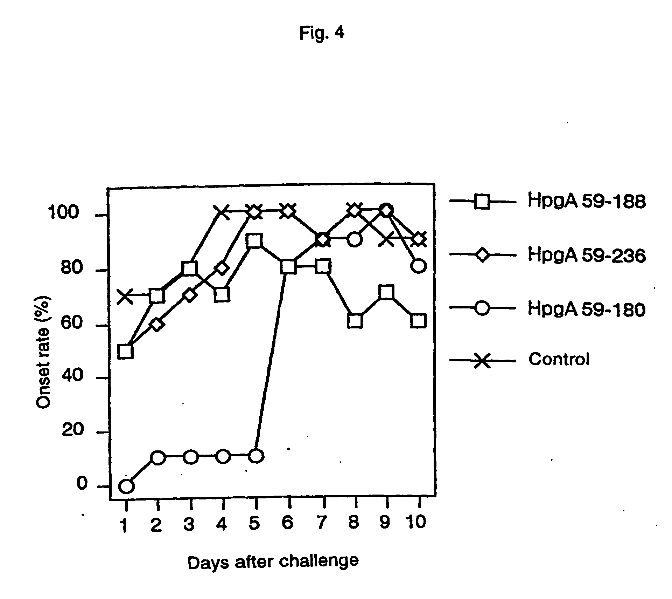

Figure 4 shows the results obtained by challenging chickens with Haemophilus paragallinarum serotype A strain 221 after passive immunization with monoclonal antibodies (clones HpgA 59-188, HpgA 59-236 and HpgA 59-180) wherein the onset of the disease was retarded in the groups previously administered with the monoclonal antibody having the HI activity (clone HpgA 59-180).

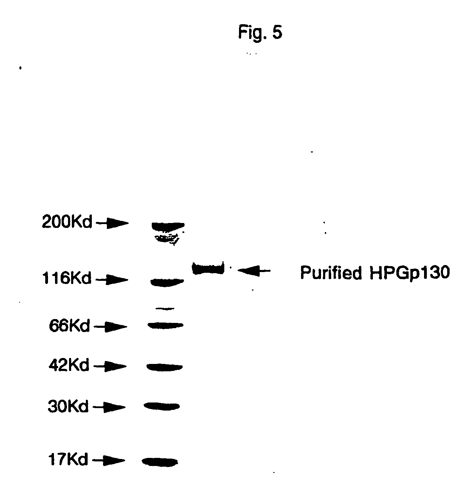

Figure 5 is a photograph showing the result of SDS-PAGE with CBB staining of HPGp130 polypeptide which is purified by affinity chromatography using the monoclonal antibody having the HI activity (clone HpgA 59-180) as a ligand.

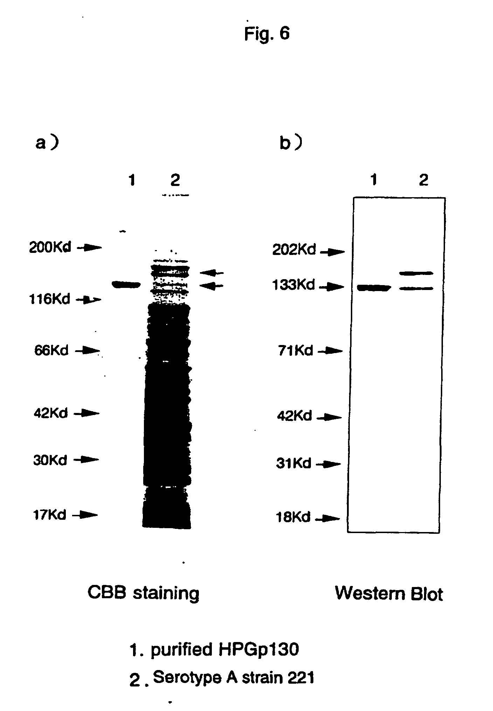

Figure 6 is (a) a photograph showing the results of SDS-PAGE with CBB staining of the purified HPGp130 polypeptide and Haemophilus paragallinarum serotype A strain 221 treated with 2-mercaptoethanol; and (b) a photograph showing the results of detection of proteins reactive with guinea pig antiserum against the purified HPGp130 polypeptide after SDS-PAGE of the purified HPGp130 polypeptide and Haemophilus paragallinarum serotype A strain 221 treated with 2-mercaptoethanol and transferring to a thin membrane (PVDF).

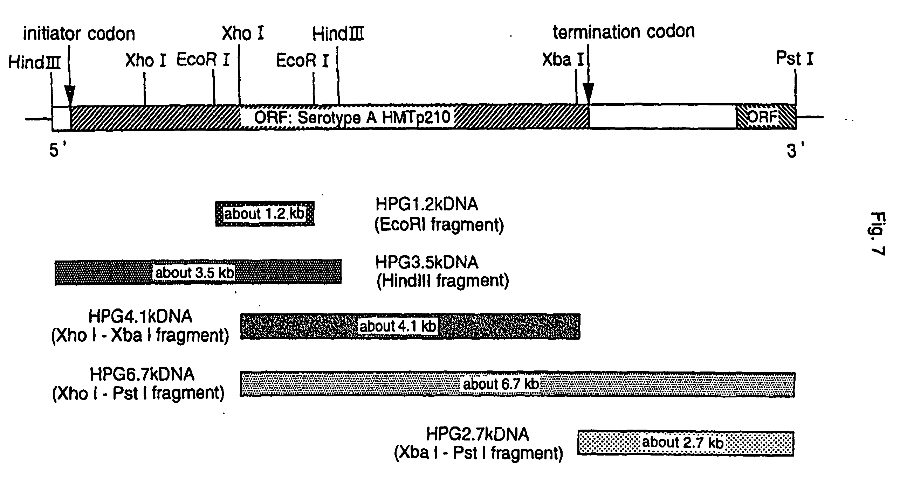

Figure 7 is a schematic illustration showing the position of HPG1.2k DNA, HPG3.5k DNA, HPG4.1k DNA, HPG6.7k DNA and HPG2.7 k DNA fragments cloned from the genome of Haemophilus paragallinarum serotype A strain 221.

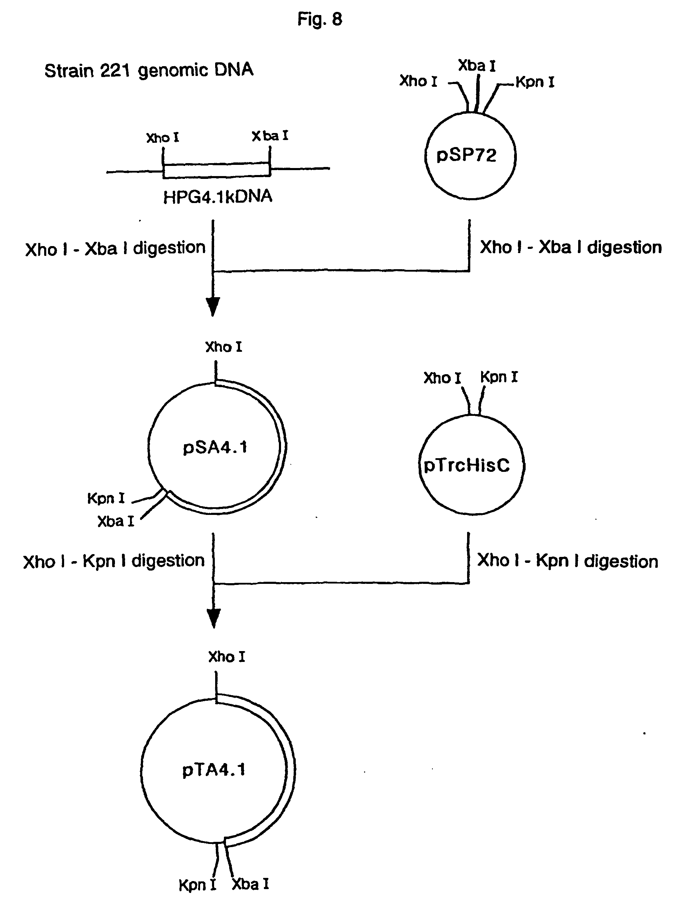

Figure 8 is a schematic illustration showing construction of plasmid pSA4.1 by inserting the XhoI-XbaI fragment (HPG4.1k DNA) from the genome of Haemophilus paragallinarum serotype A strain 221 into plasmid pSP72, followed by construction of plasmid pTA4.1 by inserting the XhoI-KpnI fragment from the plasmid pSA4.1 into plasmid pTrcHisC.

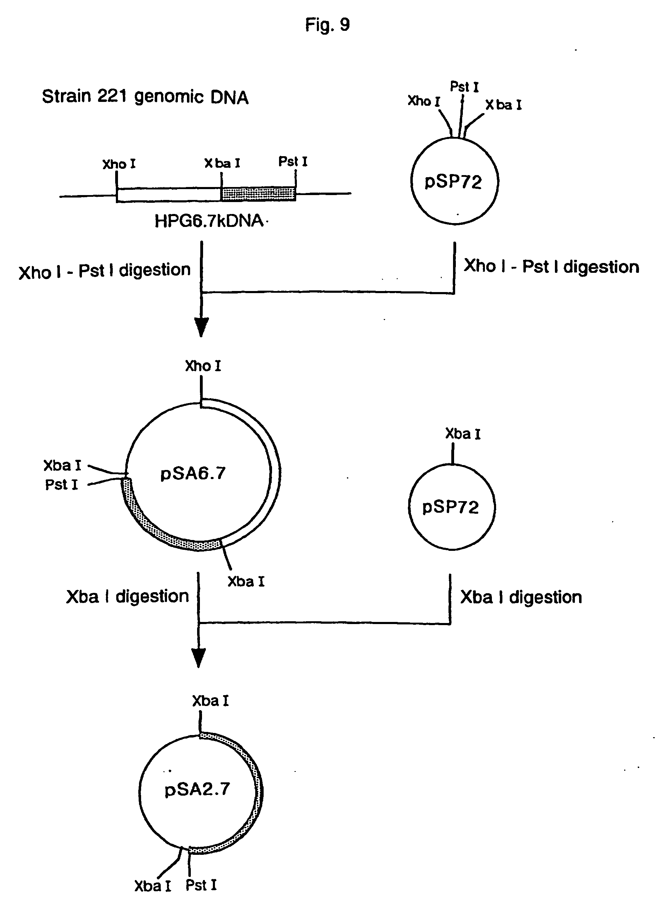

Figure 9 is a schematic illustration showing construction of plasmid pSA6.7 by inserting the XhoI-PstI fragment (HPG6.7k DNA) from the genome of Haemophilus paragallinarum serotype A strain 221 into plasmid pSP72, followed by construction of plasmid pSA2.7 by inserting the XbaI fragment from the plasmid pSA6.7 into plasmid pSP72.

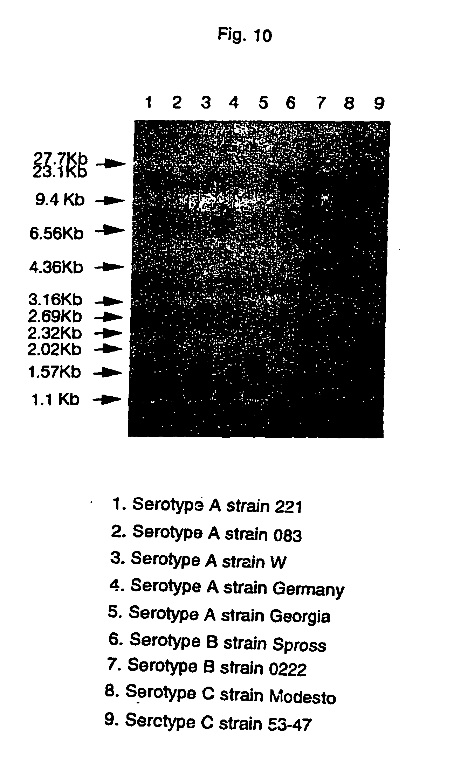

Figure 10 is a photograph showing the results of detection of DNA fragments hybridizable with HPG1.2k DNA as a probe after agarose electrophoresis of DNA fragments obtained by digesting the genome from Haemophilus paragallinarum serotypes A, B and C with restriction enzyme EcoRI and transferring to a thin membrane (Hybond N+).

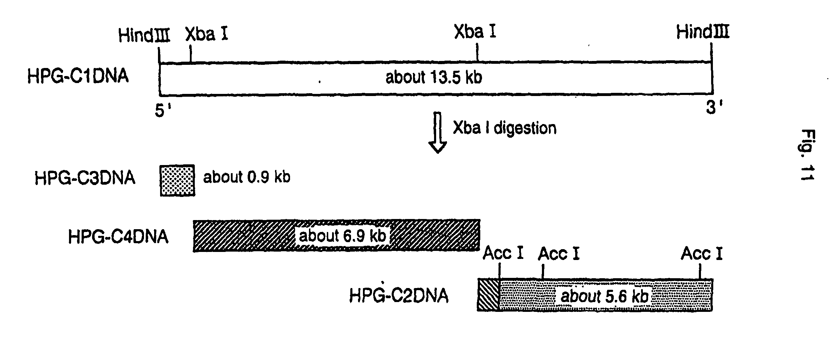

Figure 11 is a schematic illustration showing the position of HPG-C1 DNA, HPG-C2 DNA, HPG-C3 DNA and HPG-C4 DNA fragments cloned from the genome of Haemophilus paragallinarum serotype C strain 53-47.

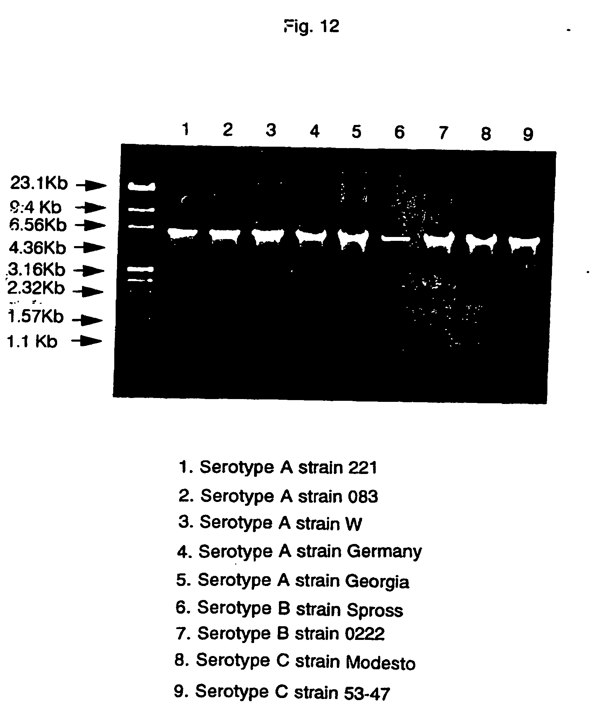

Figure 12 is a photograph showing the result of 0.8 % agarose gel electrophoresis of PCR products obtained by PCR with primers prepared on the basis of the nucleotide sequences coding for the N-terminal and C-terminal amino acid sequences of HPG serotype A HMTp210 polypeptide and the genome of Haemophilus paragallinarum serotype A, B or C as a template.

### BEST MODE FOR CARRYING OUT THE INVENTION

The present invention is explained in more detail hereinbelow.

The polypeptide from Haemophilus paragallinarum serotype A of the present invention which induces production of the HI antibody is prepared from a culture supernatant of HPG serotype A or a suspension of ruptured cells by affinity chromatography with the monoclonal antibody having the HI activity as a ligand.

The monoclonal antibody having the HI activity (hereinafter also referred to as "HI-MCA") is obtained by preparing the hybridomas producing the monoclonal antibodies which bind to Haemophilus paragallinarum serotype A by the conventional cell fusion procedure and then screening the hybridoma producing the monoclonal antibody having the HI activity with HI test.

For use as an immunogen for production of the above antibody, Haemophilus paragallinarum serotype A is obtained by the conventional procedure used for usual culture of Haemophilus paragallinarum. For example, the cells of HPG strain 221 can be recovered by shaking culture in a chicken meat infusion medium supplemented with chicken serum (including chicken meat infusion 300 ml, chicken serum 10 ml, polypeptone 5 g, glucose 1 g, casamino acid 1 g, sodium glutamate 5 g, sodium chloride 5 g, nicotinamide adenine dinucleotide 0.025 g in 1000 ml medium) at 37°C overnight followed by centrifugation.

Immunization can be carried out in a usual manner, for example, after inactivating Haemophilus paragallinarum serotype A with thimerosal, formalin and the like, by administering the inactivated cells together with the conventional adjuvant to BALB/c mouse via intraperitoneal, subcutaneous, intradermal or intravenous administration. The immunogen includes HPG cells per se, or alternatively, the cells treated with potassium rhodanide, sonication or hyaluronidase, or a processed antigen obtained by treatment with a surfactant such as sodium N-lauroylsarcosinate, Nonidet P-40 or Triton X-100. The adjuvant includes Freund's complete adjuvant, Freund's incomplete adjuvant, aluminum hydroxide gel, and the like. More specifically, immunization is conducted as follows: Haemophilus paragallinarum serotype A strain 221 cultured in a chicken meat infusion medium supplemented with chicken serum is inactivated with thimerosal and then sonicated. An emulsion obtained by mixing the resultant suspension of ruptured cells with Freund's complete adjuvant is administered subcutaneously to the back of BALB/c mouse, followed by subcutaneous administration of an emulsion prepared from the same amount of the suspension and Freund's incomplete adjuvant at the back every 2 to 4 weeks. A serum antibody level is monitored, and after confirming the elevated level of antibody titer, a suspension of ruptured cells after sonication is further administered intravenously after additional 2 to 4 weeks as a final immunization.

As an immunocyte for preparing a monoclonal antibody, splenocytes removed 2 to 4 days after the final administration is preferably used. Mouse myeloma cells include, for example, NSI-Ag4/1 (Eur. J. Immunol., 6: 511, 1976), P3X63-Ag8.U1 (Curr. Topics Microbiol. Immunol., 81:1, 1978), X63-Ag8.653 (J. Immunol., 123: 1548, 1979), and the like. Fusion of splenocytes with mouse myeloma cells may be carried out in accordance with Milstein et al., Method Enzymol., 73, 3-46,1981. That is, fusion can be carried out with approximately 1 to 10 folds higher amount of splenocytes than mouse myeloma cells. A cell fusion promoting agent may be polyethylene glycol having a molecular weight of 1,000-6,000 at a concentration of 30 to 50 % (w/v). More specifically, cell fusion is preferably carried out with about 10⁸ splenocytes and about 10⁷ P3X63-Ag8.U1 myeloma cells in a culture medium usually used for culture of lymphocytes such as RPMI 1640 medium, containing 45 % polyethylene glycol 4,000, which is previously heated at 37°C.

Hybridoma may be obtained by culture in HAT medium for a sufficient period of time so that the non-fused cells cannot survive, usually for several days to several weeks. The thus obtained hybridomas are then used for selection and cloning of strains producing a desired antibody in accordance with the usual limiting dilution procedure, using the culture supernatant of the hybridomas.

Screening of strains producing an antibody recognizing Haemophilus paragallinarum serotype A is carried out in accordance with the usual ELISA, RIA, Western blotting, and the like. An antigen used in these methods may be either a suspension of Haemophilus paragallinarum serotype A cells, the cells treated with potassium rhodanide, sonication, hyaluronidase, and the like, or an extraction of said cells with a surfactant.

Then, strains producing an antibody having the HI activity are screened in accordance with the usual HI test, using a culture supernatant of the above hybridomas or ascites from mouse administered with said hybridomas. HA antigen includes a suspension of Haemophilus paragallinarum cells or the cells treated with potassium rhodanide, sonication, hyaluronidase, and the like. Erythrocytes used for HI test may be either 0.5 % fresh chicken erythrocytes, glutaraldehyde-fixed 1 % chicken erythrocytes or formalin-fixed chicken erythrocytes, with glutaraldehyde-fixed chicken erythrocytes being preferable.

More specifically, a supernatant obtained after centrifugation of ascites treated with 5 folds amount of a 25 % kaolin solution is added to precipitates of glutaraldehyde-fixed chicken erythrocytes, which is then shaken at 37°C for 60 minutes for sensitization. To a twofold serial dilution of this supernatant is added the same amount of a suspension of strain 221 cells including 4 hemagglutinin units and the mixture is left to stand for 15 minutes. Thereto is added a suspension of glutaraldehyde-fixed 1 % chicken erythrocytes, the mixture is left to stand at room temperature for 60 minutes, and observed at the bottom of microtiter plate. An HI antibody titer is defined as a maximum dilution which can block hemagglutination.

Recovery of monoclonal antibodies having the HI activity from the thus obtained hybridomas is carried out by culturing said hybridomas in a large amount and harvesting said antibodies from the culture supernatant, or by administering said hybridomas to mice compatible with said hybridomas so that said hybridomas are proliferated and harvesting said antibodies from the ascites thereof.

Purification of the monoclonal antibody may be done by the conventional procedures used in the protein chemistry such as, for example, a salting out, ultrafiltration, an isoelectric precipitation, an electrophoresis, an ion exchange chromatography, a gel filtration chromatography, an affinity chromatography, and the like. More specifically, purification of the monoclonal antibody from ascites may be done using Protein A-Sepharose CL-4B (manufactured by Pharmacia) and MAPS-II Mouse Monoclonal Antibody Purification Kit (manufactured by Bio Rad) in accordance with protocol of the manufacturer.

An affinity column with the antibody having the HI activity as a ligand for purification of the polypeptide from Haemophilus paragallinarum serotype A which induces production of the HI antibody may be prepared by a usual procedure, for example, by binding the above purified antibody to HiTrap NHS-Activated Column (manufactured by Pharmacia) in accordance with protocol of the manufacturer.

Using the thus prepared affinity column, the polypeptide from Haemophilus paragallinarum serotype A which induces production of the HI antibody may be obtained from a culture supernatant of HPG serotype A cells or from a suspension of ruptured cells. Specifically, a polypeptide (hereinafter referred to as "HPGp130") with a molecular weight of about 130 Kd having a high capacity to produce the HI antibody and the activity to prevent avian infectious coryza was obtained from a culture supernatant of HPG strain 221 cultured in the chicken meat infusion medium supplemented with chicken serum at 37°C for two days.

An amino acid sequence of the thus obtained polypeptide may be determined by the usual procedures such as Edman degradation (P. Edman, Acta Chem. Scand., 4: 283, 1950). The amino acid sequence at the N-terminal of said polypeptide is shown in SEQ ID NO: 2.

Cloning of a gene or a gene fragment coding for the polypeptide from Haemophilus paragallinarum serotype A may be done by the usual procedures as described by Sambrook et al. (Molecular Cloning, A Laboratory Manual, 2nd Edition, Cold Spring Harbor Laboratory Press, New York, 1989). That is, Haemophilus paragallinarum serotype A strain 221 cells are cultured and recovered by the above procedures, a genomic DNA is extracted and purified with Sepagene kit (manufactured by Sanko Junyaku K.K.) in accordance with protocol attached thereto. The genomic DNA is then cleaved with a commercially available restriction enzyme (preferably EcoRI), the obtained DNA fragments are inserted into a commercially available cloning vector (e.g. λgt11) to prepare a DNA library, among which such clones expressing the antigen that responds to the desired antibody having the HI activity are screened. The antibody having the HI activity includes the culture supernatant of the hybridomas or the ascites of mice obtained as mentioned above. Antisera is preferably used which is obtained by immunization with the polypeptide from Haemophilus paragallinarum serotype A isolated by affinity chromatography with the monoclonal antibody having the HI activity as a ligand. A nucleotide sequence of the exogenous DNA fragment in the thus obtained recombinant λgt11 phage DNA may be determined with a DNA sequencer (for example, Applied Biosystems 377). Novelty of the obtained exogenous DNA fragment may be confirmed by homology search between the whole nucleotide sequence and the existing data base (for example, GeneBank, EMBL, and the like).

As shown in Example 3, for example, ten positive λgt11 phages were obtained from the DNA library and each DNA of these phages included an exogenous DNA fragment of about 1.2 kb (hereinafter also referred to as "HPG1.2k DNA fragment") as demonstrated in an agarose electrophoresis. The nucleotide sequence of said exogenous DNA corresponds to the nucleotide sequence of from nucleotides No. 1988 to No. 3157 of SEQ ID NO: 1.

Since an initiation codon and a termination codon are not found in the HPG1.2k DNA, this DNA fragment is considered to encode a portion of the polypeptide from Haemophilus paragallinarum serotype A. A gene coding for a full length of said polypeptide may be obtained by using the HPG1.2k DNA as a probe to give a longer DNA fragment, determining a nucleotide sequence of this DNA fragment and finding out an initiation codon and a termination codon.

More specifically, the genomic DNA of Haemophilus paragallinarum serotype A strain 221 is cleaved with a restriction enzyme whose cleavage site is not present in the 1.2 kb DNA (for example, HindIII) and the resulting DNA fragments are separated with an agarose electrophoresis. Using DIG-DNA Labeling Kit (manufactured by Boehringer Mannheim), Southern hybridization is carried out using digoxigenin (DIG)-labeled 1.2 kb DNA fragment as a probe for detecting desired DNA fragments. As a result, there was obtained a HindIII-digested DNA fragment of about 3.5 kb which hybridized with the 1.2 kb DNA fragment (hereinafter also referred to as "HPG3.5k DNA fragment"). A nucleotide sequence of the HPG3.5k DNA fragment corresponds to the nucleotide sequence of from nucleotides No. 1 to No. 3450 of SEQ ID NO: 1. An identical sequence to the amino acid sequence at the N-terminal of the above HPGp130 polypeptide was found at the amino acid sequence of from amino acid residues No. 1 to No. 13 (corresponding to nucleotide sequence of from No. 453 to No. 491) in SEQ ID NO: 1.

Since only an initiation codon was found in the HPG3.5k DNA but a termination codon was not, the 1.2 kb DNA fragment and the 3.5 kb DNA fragment labeled with DIG were used as a probe to give a XhoI-XbaI digested DNA fragment of about 4.1 kb (hereinafter also referred to as "HPG4.1k DNA fragment"). A nucleotide sequence of the HPG4.1k DNA fragment corresponds to the nucleotide sequence of from nucleotides No. 2212 to No. 6275 of SEQ ID NO: 1. Since a termination codon was not found in the HPG4.1k DNA fragment, a XhoI-PstI digested DNA fragment of about 6.7 kb (hereinafter also referred to as "HPG6.7k DNA fragment"; this fragment encompasses the above HPG4.1k DNA fragment) was obtained using the 1.2 kb DNA and the 3.5 kb DNA labeled with DIG. A nucleotide sequence of the HPG6.7k DNA fragment corresponds to the nucleotide sequence of from nucleotides No. 2212 to No. 8930 of SEQ ID NO: 1. There existed a termination codon in the HPG6.7k DNA fragment.

It was found that the nucleotide sequence of SEQ ID NO: 1, consisting of a total of 8930 nucleotides, included an open reading frame starting from nucleotide No. 243 which can code for 2042 amino acid residues. A polypeptide comprising the 2042 amino acid residues is hereinafter also referred to as "serotype A HMTp210". Homology search with the existing data base (GeneBank and EMBL) revealed no homology with any known nucleotide and amino acid sequences, indicating that the serotype A HMTp210 polypeptide is a novel substance.

The presence of another possible open reading frame in the nucleotide sequence of SEQ ID NO: 1 was also suggested which starts from nucleotide No. 8375 and can code for 185 amino acid residues. No termination codon was found in this sequence. Homology search with the existing data base (GeneBank and EMBL) revealed no homology with any known nucleotide and amino acid sequences, indicating that the polypeptide coded by this open reading frame is also a novel substance.

The DNA fragments from Haemophilus paragallinarum serotype A can also be used as a probe for obtaining DNA fragments from different serotype of Haemophilus paragallinarum such as serotype B or serotype C as well as polypeptides coded by said DNA fragments.

More specifically, a genomic DNA is extracted and purified from HPG serotype C strain 53-47 and cleaved with a suitable restriction enzyme (preferably HindIII), the obtained DNA fragments are inserted into a commercially available cloning vector (e.g. λDASHII) to prepare a DNA library, among which clones are screened by using the serotype A HPG3.5k DNA fragment labeled with DIG as a probe.

As shown in Example 5, ten positive λDASHII phages were obtained from the DNA library and each DNA of these phages included an exogenous DNA fragment of about 13.5 kb (hereinafter also referred to as "HPG-C1 DNA") as demonstrated in an agarose gel electrophoresis.

Since the HPG-C1 DNA fragment of about 13.5 kb is too large to be subcloned into a plasmid vector, it was cleaved with a suitable restriction enzyme (preferably XbaI) and the resulting DNA fragments were inserted into a commercially available cloning vector (for example, pUC119). As a result, DNA fragments of about 5.6 kb (hereinafter also referred to as "HPG-C2 DNA"), about 0.9 kb (hereinafter also referred to as "HPG-C3 DNA") and about 6.9 kb (hereinafter also referred to as "HPG-C4 DNA") were obtained. A nucleotide sequence of a portion of HPG-C2 DNA fragment and HPG-C4 DNA fragment was determined to reveal the presence of an initiation codon and a termination codon in these DNA fragments, respectively.

It was found that the nucleotide sequence of SEQ ID NO: 5, consisting of a total of 7486 nucleotides, included an open reading frame starting from nucleotide No. 848 which can code for 2039 amino acid residues. A polypeptide comprising the 2039 amino acid residues is hereinafter also referred to as "serotype C HMTp210". Homology search with the existing data base (GeneBank and EMBL) revealed no homology with any known nucleotide and amino acid sequences, indicating that the serotype C HMTp210 polypeptide is a novel substance.

Homology search between the nucleotide sequences coding for the serotype C HMTp210 polypeptide and the serotype A HMTp210 polypeptide revealed about 80 % homology. It was further revealed that the region of about 3.4 kb at the 5' site and the region of about 1.2 kb at the 3' site exhibited extremely high homology whereas the region of about 1.5 kb between these 5' and 3' regions showed low homology. The same was also applicable to the corresponding polypeptides encoded by these genes.

Based on the nucleotide sequence coding for the serotype A HMTp210 polypeptide, there can also be obtained, by PCR, DNA fragments from different serotype of Haemophilus paragallinarum such as serotype B or serotype C as well as polypeptides coded by said DNA fragments.

More specifically, based on the nucleotide sequence coding for the serotype A HMTp210 polypeptide, there were prepared a synthetic DNA having the nucleotide sequence of SEQ ID NO: 3 as an upstream PCR primer and a synthetic DNA having the nucleotide sequence of SEQ ID NO: 4 as a downstream PCR primer. These primers were designed such that BamHI recognition sequences were added at the 5' sites, respectively, and a full length of translation region of the serotype A HMTp210 polypeptide can be amplified. Using these primers, PCR was carried out using as a template the genomic DNAs from a total of nine strains, i.e. Haemophilus paragallinarum serotype A strains 221, 083, W, Germany and Georgia, HPG serotype B strains Spross and 0222, and HPG serotype C strains Modesto and 53-47. Analysis of the obtained PCR products on 0.8 % agarose gel electrophoresis confirmed the amplified fragment of about 6.1 kb in any of these strains.

The thus obtained DNA fragment or a portion thereof may be incorporated into a suitable expression vector, the resulting expression vector is used for transformation of a microorganism or an animal cell, and the transformant is cultured to produce the polypeptide of the present invention from Haemophilus paragallinarum or a peptide which shares at least a portion of the amino acid sequence of said polypeptide. The peptide which shares a portion of the amino acid sequence can also be produced with a peptide synthesizer.

A suitable signal sequence for secretion in a microorganism or an animal cell can also be linked upstream the DNA coding for the polypeptide of the present invention so that said polypeptide can be secreted into a culture medium. The thus modified DNA for secretion is advantageous in that said polypeptide secreted into a culture medium can easily be purified. A signal sequence includes pelB signal (S. P. Lei et al., J. Bacteriology, 169: 4379-4383, 1987) for E. coli, signal from α factor (A. J. Brake, Yeast Genetic Engineering, p269, Butterworth, 1989) for yeast, signal SG-1 from immunoglobulin (H. Maeda et al., Hum. Antibod. Hybridomas, 2: 124-134, 1991), C25 signal (PCT International Publication No. WO94/20632) for an animal cell.

An expression vector includes a plasmid, a viral vector and the like. Any promoter may be included in the expression vector such as lac, tac, pho5, adh, SV40 early, SV40 late, β actin and the like, in consideration of a microorganism or an animal cell used as a host, insofar as the polypeptide having the activity to prevent avian infectious coryza is ultimately obtained. The polypeptide of the present invention can also be expressed as a fusion protein with another protein or peptide such as β-galactosidase, glutathione-S-transferase, maltose binding protein, Protein A, histidine hexamer, and the like. A marker gene includes, in case of an expression vector for a microorganism cell, ampicillin resistant gene, tetracycline resistant gene for E. coli as a host, β-isopropyl malate dehydrogenase (Leu2) gene for yeast as a host, and in case of an expression vector for an animal cell, aminoglycoside 3' phosphotransferase (neo) gene, dihydrofolate reductase (dhfr) gene, glutamine synthetase (GS) gene, and the like. An additive for selection includes G418, neomycin, methotrexate, and the like.

Transformation of a host cell may be carried out by the known methods including, for example, a calcium chloride method, a calcium phosphate coprecipitation method, a DEAE dextran method, a lipofectin method, a protoplast polyethylene fusion method, an electroporation, and the like, which can suitably be selected depending on a host used.

The novel polypeptide of the present invention from Haemophilus paragallinarum or a peptide which shares at least a portion of the amino acid sequence of said polypeptide may be prepared as described hereinbelow. For example, the HPG3.5k DNA fragment from HGP serotype A is incorporated into an expression vector pTrcHisC (manufactured by Invitrogen), said expression vector is introduced into E. coli strain JM109 for transformation. Among the resulting transformed cells, those transformants which produce the target novel polypeptide are screened by a dot blotting with an index of reactivity with the antibody against said polypeptide. Chicken immunized with a supernatant obtained after centrifugation of a suspension of the ruptured cells have an elevated protection against challenge with HPG serotype A strain 221.

The novel polypeptide may be purified from an extract of cells or a culture supernatant from a large scale culture of the transformant producing said polypeptide by utilizing the above-mentioned methods used in the field of protein chemistry.

The thus obtained novel polypeptide from Haemophilus paragallinarum has the activity to prevent avian infectious coryza. Said polypeptide from Haemophilus paragallinarum, monoclonal and polyclonal antibodies against said polypeptide and the expression vector as mentioned above may be used as a vaccine or a therapeutic agent for avian infectious coryza either alone or in combination with a suitable carrier, diluent or stabilizing agent in a conventional manner such as injections or oral drugs.

The above novel polypeptide from Haemophilus paragallinarum or a polypeptide which shares at least a portion of the amino acid sequence of said polypeptide may be used as an immunogen for preparing a polyclonal and monoclonal antibodies in accordance with the procedures described hereinabove. Said polypeptide as well as the antibody having the capacity to bind thereto may also be utilized in an antigen or antibody detection system such as Western blot, ELISA, and the like, and may also be a material for constructing a diagnostic agent. In addition, affinity chromatography with a suitable carrier to which the above antibody is bound may be used for purification of the above polypeptide.

In accordance with the present invention, there are provided the novel polypeptide from Haemophilus paragallinarum and the gene fragment coding for said polypeptide for prevention of avian infectious coryza and the antibody having the HI activity which can be used as a therapeutic agent.

The polypeptide from Haemophilus paragallinarum, which the present inventor has found, has a molecular weight of about 130 Kd, has the activity to induce production of the HI antibody, and is the novel, important polypeptide for prevention of avian infectious coryza. Technical problems associated with the obtention of said polypeptide, such as isolation of the gene coding for said polypeptide, construction of the expression vector, preparation of the expression cell, and purification of said polypeptide, are solved by the present invention, which allows for provision of a more effective vaccine than the prior art vaccines. Furthermore, these are useful as a material for providing a rapid, simple diagnostic agent for avian infectious coryza.

The present invention is illustrated in more detail by means of the following Examples but should not be construed to be limited thereto.

### Example 1: Preparation and features of monoclonal antibody

### (1) Preparation of monoclonal antibody

Haemophilus paragallinarum serotype A strain 221 cells were inoculated to 100 ml of chicken meat infusion medium supplemented with chicken serum and shake-cultured at 37°C overnight, followed by centrifugation (8,000 rpm, 20 minutes) to recover cells. The obtained cells were washed with PBS while centrifugation and then suspended in PBS containing 0.01 % thimerosal at about 5 x 10¹⁰ cells/ml. The suspension was sonicated with Branson Sonifier 350 at 20 kHz, 4°C for 10 minutes (alternative repeat of sonication for 0.5 second and cooling for 0.5 second). The thus obtained suspension of the ruptured cells by sonication was mixed with the same amount of Freund's complete adjuvant and the mixture was well blended till a water-in-oil (w/o) was achieved. Each 0.1 ml of this emulsion was subcutaneously administered to BALB/c mouse at two sites of the back. Four weeks later, each 0.1 ml of an emulsion prepared similarly with Freund's incomplete adjuvant was subcutaneously administered at two sites of the back. After additional 18 days, 0.1 ml of the suspension of the ruptured cells by sonication was intravenously administered.

Three days after the final administration, splenocytes were removed. Said splenocytes (1 x 10⁸ cells) were mixed with mouse myeloma cells P3X63-Ag8.U1 (1 x 10⁷ cells) by padding, thereto was added RPMI1640 medium containing 45 % polyethylene glycol previously warmed at 37°C to conduct cell fusion. The cells after fusion reaction were suspended in HAT medium (RPMI1640 medium containing 5 % fetal calf serum supplemented with 1 x 10⁻⁴ M hypoxanthine, 4 x 10⁻⁷ M aminopterin and 1.6 x 10⁻⁵ M thymidine), and after plated on 96-well microtiter plate for cell culture (manufactured by Coning), cultured under the condition of 37°C and 5 % CO₂.

For the wells where hybridomas propagated, the presence of the monoclonal antibody recognizing Haemophilus paragallinarum in the culture supernatant was determined with ELISA as described hereinbelow. A suspension of ruptured cells by sonication of Haemophilus paragallinarum serotype A strain 221 prepared as mentioned above was diluted 300 folds with PBS and each 100 µl of the suspension was plated on well of microtiter plate for ELISA (Immulon II manufactured by Dynatech). The microtiter plate was left to stand at 4°C overnight and masked with PBS containing 5 % skim milk at 200 µl per well at room temperature for 2 hours. The microtiter plate was washed with PBS containing 0.05 % Tween 20 (PBS-T) and thereto was added 100 µl of the culture supernatant of hybridomas diluted 10 folds with PBS-T containing 5 % skim milk for reaction at room temperature for 2 hours. After washing with PBS-T, each 100 µl of peroxidase-labeled antimouse IgG (manufactured by Bio-Rad) diluted 10,000 folds with PBS-T containing 5 % skim milk was added for reaction at room temperature for 2 hours. Then, after washing with PBS-T, each 100 µl of 0.05 M citrate-0.1 M disodium hydrogenphosphate buffer (pH 5.0) containing 6 mg per 11 ml of ortho-phenylenediamine dihydrochloride (OPD; manufactured by Katayama Kagaku K.K.) and 4.75 µl of hydrogen peroxide (containing H₂O₂ at 31 %; manufactured by Mitsubishi Gasu Kagaku K.K.) was added for reaction at room temperature for 30 minutes. Each 50 µl of 3 M sulfuric acid was added to quench the reaction and absorbance (490 nm) of each well was measured with Autoreader for ELISA.

Hybridomas of the wells where the antibody against Haemophilus paragallinarum serotype A was secreted in the culture supernatant were cloned by a limiting dilution method so that they become monoclonal. Thus, nine clones producing the monoclonal antibody against Haemophilus paragallinarum serotype A were obtained.

### (2) HI activity of monoclonal antibodies

These hybridomas were cultured in a large amount and intraperitoneally administered to BALB/c mice, pretreated with an immunosuppressive agent, pristane (2,6,10,14-tetramethylpentadecane; manufactured by Aldrich), where the hybridomas propagated. Ten to twenty days later, the mice were sacrificed and the produced ascites were removed therefrom and HI activity of the ascites was determined.

A suspension of Haemophilus paragallinarum serotype A strain 221 cells inactivated with thimerosal was used as an HA antigen for HI test and prepared based on HA titer. First, using a V-shaped microtiter plate (Sanko Junyaku K.K.), a suspension of a glutaraldehyde-fixed 1 % chicken erythrocytes (0.05 ml) was added to a 2 folds serial dilution of HA antigen (0.05 ml), and after standing at room temperature for 60 minutes, the bottom of the plate was observed. A maximum dilution which agglutinates erythrocytes was defined as HA titer and, regarding a concentration of HA antigen at this dilution as 1 unit, a stock solution of HA antigen was prepared so that it contains 4 units.

Then, to 0.2 ml of mouse ascites was added 5 folds amount of 25 % kaolin solution and the mixture was shaken at 37°C for 30 minutes for sensitization, followed by centrifugation to give a supernatant. This supernatant of centrifugation after kaolin treatment was added to precipitates obtained by centrifugation of glutaraldehyde-fixed 10 % chicken erythrocytes (2 ml) and the mixture was shaken for sensitization at 37°C for 60 minutes. After sensitization, a supernatant was obtained by centrifugation and used as 5 folds diluted mouse ascites for determination of HI antibody. Using a V-shaped microtiter plate, to 0.025 ml of a 2 folds serial dilution of this supernatant was added the same amount of the suspension of strain 221 cells inactivated with thimerosal containing 4 hemagglutination units and, after mixing, the mixture was left to stand for 15 minutes. After sufficient sensitization, 0.05 ml of a suspension of glutaraldehyde-fixed 1 % chicken erythrocytes was added. After the mixture was left to stand at room temperature for 60 minutes, the bottom of the microtiter plate was observed. A maximum dilution which inhibits hemagglutination was defined as an HI antibody titer. Among nine clones, the monoclonal antibodies from three clones (HpgA 59-40, HpgA 59-145 and HpgA 59-180) exhibited a high HI activity (Table 1). The clone HpgA 59-180 has been deposited by the applicant as FERM BP-6084 at National Institute of Bioscience and Human-Technology Agency of Industrial Science and Technology (1-3, Higashi 1-chome, Tsukuba-shi, Ibaraki-ken) on September 5, 1996.

**Table 1**

| Monoclonal antibody | HI antibody titer |
|---|---|
| HpgA 59-33 | <50 |
| HpgA 59-40 | 25,600 |
| HpgA 59-48A | <50 |
| HpgA 59-48B | <50 |
| HpgA 59-145 | 1,600 |
| HpgA 59-180 | 12,800 |
| HpgA 59-188 | <50 |
| HpgA 59-236 | <50 |
| HpgA 59-284 | <50 |

### (3) Protective activity of monoclonal antibodies

A mouse ascites (0.3 ml) containing these antibodies was intraperitoneally administered to SPF white leghorn chickens of 4 to 6 weeks old, each group comprising 8 to 10 chickens, and on the next day, about 10⁸ cells of Haemophilus paragallinarum serotype A strain 221 were applied dropwise to the nasal cavity of the chickens for challenge. A control group which was given no mouse ascites was also used and was challenged in the same manner. Each group was observed for the presence of the coryza symptoms (i.e. a running nose, swelling of the face and epiphora) for 10 days. All the groups which previously received the monoclonal antibodies having the HI activity (hereinafter also referred to as "HI-MCA") were likely to retard the onset as compared to the control group. On the contrary, all the groups administered with the monoclonal antibodies of the other clones showed no significant difference (Figs. 1 to 4).

### Example 2: Purification and property of antigen recognized by HI-MCA

### (1) Purification of HI-MCA

HI-MCA (HpgA 59-180) was purified from mouse ascites using Protein A-Sepharose CL-4B (manufactured by Pharmacia) and MAPS-II Mouse Monoclonal Antibody Purification Kit (manufactured by Bio-Rad) in accordance with protocol attached thereto. First of all, to 4 ml of mouse ascites was added the same amount of a binding buffer included in the Antibody Purification Kit. After the mixture was filtered with Sterivex filter of 0.45 micron (manufactured by Millipore), it was applied to Protein A-Sepharose CL-4B column (gel bed volume 5 ml) and was thoroughly washed with the binding buffer till less than 0.05 of the absorbance at 280 nm was obtained. Then, the antibodies bound to the column were eluted with an elution buffer included in the kit. The eluted antibodies were dialyzed against 0.2 M sodium hydrogen carbonate (pH 8.3) containing 0.5 M sodium chloride to give 40 mg of purified HI-MCA (HpgA 59-180). Similarly, HI-MCA (HpgA 59-40) was also purified to give 12 mg.

### (2) Binding of HI-MCA to carrier

Then, the purified HI-MCA (HpgA 59-180) as a ligand was bound to HiTrap NHS-activated column (manufactured by Pharmacia) in accordance with protocol attached thereto. First of all, HiTrap NHS-activated column (gel bed volume 1 ml) was washed with 1 mM hydrochloric acid and then circulated with 0.2 M sodium hydrogen carbonate solution (10 ml) containing 0.5 M sodium chloride and 10 mg of the above purified HI-MCA (HpgA 59-180) at room temperature for 30 minutes so that HI-MCA was bound to the column. The obtained HI-MCA-bound HiTrap column was washed each three times alternatively with 0.5 M ethanolamine (pH 8.3) containing 0.5 M sodium chloride, and 0.1 M sodium acetate buffer (pH 4.0) containing 0.5 M sodium chloride and equilibrated with PBS for purification of an antigen recognized by HI-MCA.

### (3) Purification of antigen recognized by HI-MCA

An antigen was purified from a culture of Haemophilus paragallinarum serotype A strain 221 by an affinity chromatography using HI-MCA as a ligand. An antigen was detected by ELISA method as described hereinbelow.

The above purified HI-MCA (HpgA 59-40) was diluted with 0.05 M sodium carbonate buffer (pH 9.0) to a concentration of 1.6 µg/ml and was placed in a well of microtiter plate for ELISA. The plate was left to stand at 4°C overnight and masked with PBS containing 5 % skim milk at room temperature for 2 hours. After washing with PBS-T, an eluate from the column diluted 10 folds with PBS-T containing 5 % skim milk was reacted at room temperature for 2 hours. After washing with PBS-T, peroxidase-labeled HI-MCA (HpgA 59-180) diluted 10,000 folds with PBS-T containing 5 % skim milk was reacted at room temperature for 2 hours. Then, after washing with PBS-T, a substrate solution containing OPD and hydrogen peroxide was added for reaction at room temperature for 30 minutes. Peroxidase-labeled HI-MCA (HpgA 59-180) was prepared by binding horseradish peroxidase (manufactured by Toyobo K.K.) to the above purified HI-MCA (HpgA 59-180) as described by Yoshitake et al. (J. Biochem., 92: 1413-1424, 1982).

Haemophilus paragallinarum serotype A strain 221 cells were inoculated to 100 ml of chicken meat infusion culture supplemented with chicken serum and shake-cultured at 37°C for 2 days. To a culture supernatant obtained after removal of cells by centrifugation at 8,000 rpm for 20 minutes was immediately added a serine protease inhibitor, phenylmethylsulfonyl fluoride, at 1 mM, and the mixture was filtered with 0.45 micron Sterivex filter. The HI-MCA-bound HiTrap column preequilibrated with PBS was added with 60 ml of the above filtrate and washed with PBS. When the absorbance at 280 nm became less than 0.05, an antigen bound to HI-MCA was eluted with 3M sodium thiocyanate. Antigens recognized by HI-MCA were not found in unbound fractions but in most part were recovered in fractions eluted with 3 M sodium thiocyanate. This eluate was dialyzed against 50 mM Tris-HCl buffer (pH 8.0) containing 50 mM sodium chloride. (4) Amino acid sequence analysis of N-terminal of antigen recognized by HI-MCA

After treatment with 2-mercaptoethanol, the eluate from the affinity column was subjected to sodium dodecyl sulfate polyacrylamide gel electrophoresis (SDS-PAGE) with 5 to 20 % polyacrylamide gel in accordance with Laemmli, Nature, 227: 680-685, 1970, which was stained with 0.25 % Coomassie Brilliant Blue R250 (CBB) dissolved in 50 % methanol - 10 % acetic acid to reveal a band of a molecular weight about 130 Kd (Fig. 5). This polypeptide was referred to as HPGp130 and an amino acid sequence of the N-terminal was determined as described hereinbelow.

First, the purified HPGp130 polypeptide was treated with 2-mercaptoethanol and then subjected to SDS-PAGE using 5 % polyacrylamide gel. After electrophoresis, the gel was washed with a transfer buffer (10 mM N-cyclohexyl-3-aminopropanesulfonic acid, 10 % methanol, pH 11) and overlaid to polyvinylidene difluoride (PVDF) membrane (manufactured by Millipore), which was previously immersed successively in 100 % methanol and a transfer buffer, followed by transfer with TRANS-BLOT CELL (manufactured by Bio Rad) at 20 V overnight. The PVDF membrane after transfer was washed with water and stained with 0.1 % Amido Black dissolved in 45 % methanol - 10 % acetic acid for 30 seconds, followed by decolorization with distilled water.

The stained band of a molecular weight 130 Kd was cut out and analyzed with Protein Sequencer (Applied Biosystems 477A). Thirteen amino acid residues at the N-terminal were analyzed, and as a result, the amino acid sequence was found to be Lys-Trp-Leu-Glu-Val-Tyr-Ser-Ser-SerVal-Lys-Leu-Ser as shown in SEQ ID NO: 2.

### (5) Induction of HI antibody production by HPGp130

Whether HPGp130 polypeptide could induce production of HI antibody was investigated. An emulsion (1 ml; about 20 µg of HPGp130 polypeptide per animal) prepared by mixing the HPGp130 polypeptide solution (about 40 µg/ml) with the same amount of Freund's complete adjuvant was subcutaneously injected to guinea pig at two sites of the back for immunization. About three weeks later, 1 ml of an emulsion prepared similarly with Freund's incomplete adjuvant was injected subcutaneously at two sites of the back. Additional two weeks later, the emulsion prepared with Freund's incomplete adjuvant was boosted subcutaneously at two sites of the back and four weeks thereafter the test animals were bled. HI antibody titer of the obtained antisera was determined as described above to reveal a high HI antibody titer (5,120 folds). Thus, it was found that the HPGp130 polypeptide induced production of HI antibody deeply involved in protection against avian infectious coryza.

### (6) Peptide recognized by anti-HPGp130 polypeptide guinea pig sera

A polypeptide recognized by anti-HPGp130 polypeptide guinea pig serum was analyzed by Western blot. First, the purified HPGp130 polypeptide and HPG serotype A strain 221 cells cultured in chicken meat infusion medium supplemented with chicken serum were treated with 2-mercaptoethanol and subjected to SDS-PAGE. After completion of electrophoresis, the gel was immersed in a transfer buffer (25 mM Tris, 192 mM glycine, 20 % ethanol, pH 8.3) for 5 minutes and overlaid to PVDF membrane, which was previously immersed in 100 % methanol and the transfer buffer in this order, and a transfer was carried out using TRANS-BLOT SD CELL (manufactured by Bio Rad) at 7 V for 1 hour. The membrane was masked with PBS containing 5 % skim milk at 4°C overnight, washed with PBS-T and then reacted with anti-HPGp130 polypeptide guinea pig serum diluted 1,000 folds with PBS-T containing 5 % skim milk at room temperature for 2 hours. After washing with PBS-T, peroxidase-labeled anti-guinea pig IgG (manufactured by Zymed) diluted 2,000 folds with PBS-T containing 5 % skim milk was reacted at room temperature for 2 hours. After washing with PBS-T, the membrane was immersed in 10 ml of 0.1 M Tris-HCl buffer (pH 7.5) containing 5 mg of 3,3'-diaminobenzidine tetrahydrochloride (DAB; manufactured by Dojin Kagaku K.K.) and 3 µl of hydrogen peroxide for reaction. As a result, anti-HPGp130 polypeptide guinea pig serum recognized the HPGp130 polypeptide and a band of a molecular weight about 160 Kd, possibly a precursor of the polypeptide (Fig. 6).

### (7) Immunogenicity of HPGp130 polypeptide

In accordance with the procedures as described hereinabove, ten SPF white leghorn chickens of 5 weeks old were immunized by subcutaneously administering at the leg 0.5 ml of an emulsion (containing about 10 µg of HPGp130 polypeptide) prepared by mixing an HPGp130 polypeptide solution (about 40 µg/ml) and the same amount of Freund's complete adjuvant. Three weeks later, the chickens were subcutaneously administered at the leg with 0.5 ml of an emulsion prepared similarly with Freund's incomplete adjuvant. Two weeks later, the chickens were boosted subcutaneously at the leg with an emulsion prepared similarly with Freund's incomplete adjuvant. Seven weeks after the first immunization, the chickens were challenged with Haemophilus paragallinarum serotype A strain 221. As a control, one group was immunized twice with 0.5 ml of 0.25 % formalin-inactivated HPG serotype A strain 221 (cell number prior to inactivation: 4 x 10⁸ cells/ml) supplemented with aluminum hydroxide gel (in terms of aluminum: 0.5 mg/ml) at the interval of three weeks and another group was not immunized and both control groups were challenged similarly. The results are shown in Table 2. Both groups immunized either with HPGp130 polypeptide or formalin-inactivated cells showed protection against the onset of the disease in all the chickens. For the non-immunization group, however, the symptoms were shown in all the chickens.

**Table 2**

| Immunization group | Tested chicken | Protected chicken | Protection rate (%) |
|---|---|---|---|
| Purified HPGp130 | 10 | 10 | 100 |
| Formalin-inactivated strain 221 | 10 | 10 | 100 |
| Non immunization control | 8 | 0 | 0 |

### Example 3: Cloning of gene coding for polypeptide (serotype A HMTp210) from Haemophilus paragallinarum serotype A strain 221

### (1) Screening from genomic library

Haemophilus paragallinarum serotype A strain 221 cells were inoculated to 5 ml of chicken meat infusion medium supplemented with chicken serum and shake-cultured at 37°C overnight and the cells were recovered by centrifugation. After washing the obtained cells with PBS by centrifugation, DNA was extracted and purified from the cells with Sepagene kit (manufactured by Sanko Junyaku K.K.) in accordance with protocol attached thereto. The DNA was dissolved in 50 µl of TE buffer (10 mM Tris-HCl buffer containing 1 mM EDTA, pH 8.0) and the obtained solution was used as a genomic DNA solution. Then, using cDNA Rapid Cloning Module-λgt11 (manufactured by Amersham), 0.2 µg of the genomic DNA digested with restriction enzyme EcoRI was ligated to 0.5 µg of λgt11 arm digested with restriction enzyme EcoRI in accordance with protocol attached thereto. Using λ-DNA In Vitro Packaging Module (manufactured by Amersham), the ligated product was inserted into λ phage in accordance with protocol attached thereto. The obtained solutions of recombinant phage were used as a genomic library.

The above solutions of genomic library were added to a suspension of E.coli strain Y1090 (manufactured by Amersham) about 10⁸ cells in an aqueous solution of 10 mM magnesium sulfate for absorption at 37°C for 15 minutes. Thereto was added LB soft agar medium (containing tryptone 10 g, yeast extract 5 g, sodium chloride 10 g, ampicillin 50 mg, maltose 4 g and agar 8 g in 1000 ml, pH 7) for overlay warmed at 45°C. The mixture was overlaid to LB agar medium (containing tryptone 10 g, yeast extract 5 g, sodium chloride 10 g, ampicillin 50 mg and agar 15 g in 1000 ml, pH 7) and incubated at 42°C for 3 hours. A nitrocellulose membrane immersed in an aqueous solution of 10 mM isopropyl-β-D-thiogalactopyranoside (IPTG) was air-dried, overlaid to the above plate and incubated at 37°C overnight. The nitrocellulose membrane was then peeled off from the plate, washed with PBS-T and masked with PBS containing 5 % skim milk at room temperature for 2 hours. Thereafter, the procedures as described in Example 2 (6) were repeated so that anti-HPGp130 polypeptide guinea pig serum, peroxidase-labeled anti-guinea pig IgG and a substrate were successively reacted. A series of these procedures gave plaques which express an antigen specifically reactive with anti-HPGp130 guinea pig serum from Haemophilus paragallinarum serotype A strain 221. About 5,000 plaques were immunologically screened as described above to give 43 positive plaques. These positive plaques were recovered in an SM buffer (50 mM Tris-HCl buffer containing 0.1 M sodium chloride, 10 mM magnesium sulfate and 0.01 % gelatin, pH 7.5) and, after adding several drops of chloroform, stored at 4°C. Ten among the recovered positive plaques were further subjected to second and third screening as in the primary screening.

The recombinant λgt11 phages found positive in the immunological screening were added to a suspension of E. coli strain Y1090 about 10⁸ cells in an aqueous solution of 10 mM magnesium sulfate for absorption at 37°C for 15 minutes. Thereto was added 10 ml of LB liquid medium containing 0.4 % maltose, 5 mM calcium chloride and ampicillin 50 µg/ml and the cells were further cultured at 37°C overnight. After bacteriolysis with addition of several drops of chloroform, the lysis solution was centrifuged to remove the intact E. coli cells and debris. To 5 ml of the obtained culture supernatant was added the same amount of an aqueous solution of 2.5 M sodium chloride containing 20 % polyethylene glycol 6,000 and the mixture was left to stand on ice for 1 hour. After centrifugation at 10,000 rpm, precipitated λgt11 phage was subjected to phenol treatment and isopropanol precipitation to recover phage DNA. About 150 µg of the obtained phage DNA was digested with EcoRI and then electrophoresed on 0.8 % agarose gel to separate DNA fragments derived from Haemophilus paragallinarum serotype A strain 221. Using Sephaglas™ BandPrep Kit (manufactured by Pharmacia), the DNA fragments were eluted and recovered from the gel in accordance with protocol attached thereto. All the DNA fragments obtained from ten positive phages had a length of about 1.2 kb. A DNA fragment (hereinafter referred to as "HPG1.2k DNA") obtained from the phage of a clone (clone 2) was used in the following test.

### (2) Nucleotide sequence of HPG1.2k DNA fragment

Plasmid pUC119 (manufactured by Takara Shuzo K.K.) was digested with EcoRI and then treated with alkaline phosphatase to dephosphorize the 5' end. The cleaved pUC119 DNA was treated with phenol and chloroform and then harvested by precipitation with ethanol. The cleaved pUC119 and the HPG1.2k DNA fragment were ligated together with DNA Ligation Kit ver. 2 (manufactured by Takara Shuzo K.K.). Competent cells of E.coli strain JM109 (manufactured by Takara Shuzo K.K.) were transformed with the ligated product and then cultured on Circle Grow agar medium (manufactured by BIO101) containing 50 µg/ml of ampicillin at 37°C overnight. Colonies grown on the agar medium were inoculated to 0.5 ml of Circle Grow medium containing 50 µg/ml of ampicillin and cultured at 37°C for 5 hours. Plasmids were extracted from the cells by an alkali method and, after digestion with EcoRI, subjected to 0.8 % agarose gel electrophoresis to detect recombinant plasmids containing DNA fragment with the same length as the 1.2k DNA derived from Haemophilus paragallinarum serotype A strain 221, and thereby transformed E.coli were confirmed.

The obtained transformants of E.coli were cultured on Circle Grow medium containing 50 µg/ml of ampicillin and then the recombinant plasmids (hereinafter referred to as "pUA1.2") were recovered from the cells by PEG precipitation method. Using a Primer Walking method, a nucleotide sequence of the HPG1.2k DNA fragment was analyzed using a DNA sequencer (Applied Biosytems 377). As a result, a sequence of 1170 nucleotides was determined. It was found that the nucleotide sequence of the HPG1.2k DNA corresponds to the sequence of from No. 1988 to No. 3157 in SEQ ID NO: 1, which is a nucleotide sequence coding for serotype A HMTp210 polypeptide as described hereinbelow, and codes for 389 amino acid residues with no initiation codon and termination codon within this region. A corresponding amino acid sequence was also shown which depicts no sequence equivalent to the N-terminal amino acid sequence of HPGp130 polypeptide. Accordingly, it was considered that HPG1.2k DNA codes for a portion of HPGp130 polypeptide.

### (3) Cloning of HPG3.5k DNA

Using DIG-DNA Labeling Kit (manufactured by Boehringer Mannheim), about 0.3 µg of the above HPG1.2k DNA was labeled with digoxigenin (DIG) in accordance with protocol attached thereto. After the genomic DNA of Haemophilus paragallinarum serotype A strain 221 was cleaved with several restriction enzymes, a suitable amount of the cleaved products was electrophoresed on 0.8 % agarose gel and then transferred to Hybond N+ membrane (manufactured by Amersham). Using the DIG-labeled HPG1.2k DNA as a probe, a Southern hybridization was carried out with DIG Nucleic Acid Detection Kit (manufactured by Boehringer Mannheim) in accordance with protocol attached thereto for detection of desired DNAs. As a result, about 3.5 kb fragment obtained by HindIII digestion hybridized to the DIG-labeled HPG1.2k DNA. Thus, this fragment was separated on 0.8 % agarose gel electrophoresis and eluted and recovered from the gel with Sephaglas™ BandPrep Kit in accordance with protocol attached thereto.

On the other hand, plasmid pUC119 was digested with HindIII and then treated with alkaline phosphatase to dephosphorize the 5' end. The cleaved pUC119 DNA was treated with phenol and chloroform and then recovered by precipitation with ethanol. The cleaved pUC119 and the above HindIII digest (about 3.5 kb) from the genome of Haemophilus paragallinarum serotype A strain 221 were ligated together with DNA Ligation Kit ver. 2. Competent cells of E.coli strain JM109 were transformed with the ligated product and then cultured on Circle Grow agar medium containing 50 µg/ml of ampicillin at 37°C overnight. To the agar medium where transformed E.coli grown was overlaid Hybond N+ membrane to lift the colonies. Using the DIG-labeled HPG1.2k DNA as a probe a colony hybridization was carried out in the conventional manner and positive clones were screened with DIG Nucleic Acid Detection Kit.

The positive clones were cultured on Circle Grow medium containing 50 µg/ml of ampicillin. Plasmids were recovered from the cells by PEG precipitation method. The obtained recombinant plasmid (hereinafter referred to as "pUA3.5") was digested with HindIII and then electrophoresed on 0.8 % agarose gel to separate 3.5 kb DNA fragment derived from Haemophilus paragallinarum serotype A strain 221. Using Sephaglas™ BandPrep Kit, this DNA fragment (hereinafter referred to as "HPG3.5k DNA") was eluted and recovered in accordance with protocol attached thereto. E.coli UA3.5JM transformed with the recombinant plasmid has been deposited by the applicant as FERM BP-6083 at National Institute of Bioscience and Human-Technology Agency of Industrial Science and Technology (1-3, Higashi 1-chome, Tsukuba-shi, Ibaraki-ken) on September 5, 1996.

### (4) Expression of HPG3.5k DNA

The expression vector pTrcHisC (manufactured by Invitrogen) was digested with HindIII and then treated with alkaline phosphatase to dephosphorize the 5' end. The cleaved pTrcHisC DNA was treated with phenol and chloroform and then recovered by precipitation with ethanol. The cleaved pTrcHisC and the above HPG3.5k DNA were ligated together with DNA Ligation Kit ver. 2. Competent cells of E.coli strain JM109 were transformed with the ligated product and then cultured on Circle Grow agar medium containing 50 µg/ml of ampicillin at 37°C overnight. Colonies grown on the agar medium were inoculated to 0.5 ml of Circle Grow medium containing 50 µg/ml of ampicillin and cultured at 37°C for 5 hours. Plasmids were extracted from the cells by an alkali method and, after digestion with HindIII, subjected to 0.8 % agarose gel electrophoresis to detect recombinant plasmids containing DNA fragment with the same length as the 3.5k DNA derived from Haemophilus paragallinarum serotype A strain 221, and thereby transformed E.coli cells were confirmed.

The obtained transformants of E.coli were plated on 1 ml of Circle Grow medium containing 50 µg/ml of ampicillin and cultured at 37°C for 3 hours. Thereto was further added IPTG (final concentration of 1 mM) and the transformants were cultured at 37°C for additional 3 hours. The cells were harvested from the culture by centrifugation and suspended in 50 µl of PBS. The suspension of the cells (10 µl) was mixed with the same amount of 2 % SDS and the mixture was boiled for 5 minutes and 2 µl was then spotted on a nitrocellulose membrane. The nitrocellulose membrane was air-dried and then masked with PBS containing 5 % skim milk at 4°C overnight. Thereafter, the procedures as described in Example 2 (6) were repeated so that anti-HPGp130 polypeptide guinea pig serum, peroxidase-labeled anti-guinea pig IgG and a substrate were successively reacted. A series of these procedures gave E.coli which was transformed with a recombinant plasmid wherein HPG3.5k DNA was ligated in a right direction and expresses an antigen specifically reactive with anti-HPGp130 guinea pig serum.

### (5) Immunogenicity of HPG3.5k-HIS polypeptide

The obtained transformants of E.coli were inoculated to 200 ml of Circle Grow medium containing 50 µg/ml of ampicillin and cultured at 37°C for 3 hours. Thereto was added IPTG (final concentration of 1 mM) and the transformants were cultured at 37°C for additional 3 hours. The cells were harvested from the culture by centrifugation and suspended in 10 ml of PBS. To the suspension was added lysozyme at 100 µg/ml for reaction at 4°C for 1 hour. The suspension was sonicated with Branson Sonifier 350 at 4°C for 10 minutes for bacteriolysis. Intact cells were removed by centrifugation and the obtained supernatant was used as a crude HPG3.5k-HIS polypeptide.

Ten SPF white leghorn chickens of 8 weeks old were immunized by subcutaneously administering at the leg 0.5 ml of an emulsion prepared by thoroughly mixing the crude HPG3.5k-HIS polypeptide solution with the same amount of Freund's complete adjuvant. Three weeks later, the chickens were subcutaneously administered at the leg with 0.5 ml of an emulsion prepared similarly with Freund's incomplete adjuvant. Two weeks later, the chickens were boosted subcutaneously at the leg with an emulsion prepared similarly with Freund's incomplete adjuvant. Seven weeks after the first immunization, the chicken were challenged with Haemophilus paragallinarum serotype A strain 221. As a control, as described in Example 2 (7), one group was immunized with formalin-inactivated HPG serotype A strain 221 and another group was not immunized and both control groups were challenged similarly. The results are shown in Table 3. The group immunized with the crude HPG3.5k-HIS polypeptide showed protection against the onset of the disease in seven among ten chicken. The group immunized with the formalin-inactivated cells exhibited protection against the onset of the disease in all the chickens whereas the non-immunization group showed the symptoms in all the chickens.

**Table 3**

| Immunization group | Tested chicken | Protected chicken | Protection rate (%) |
|---|---|---|---|
| Crude HPGp3.5k-HIS | 10 | 7 | 70 |
| Formalin-inactivated strain 221 | 10 | 10 | 100 |
| Non immunization control | 8 | 0 | 0 |

### (6) Nucleotide sequence of HPG3.5k DNA fragment

A nucleotide sequence of HPG3.5k DNA fragment was analyzed with a DNA sequencer as described above. As a result, a sequence of 3450 nucleotides was determined. The nucleotide sequence of HPG3.5k DNA fragment corresponds to the nucleotide sequence of from nucleotides No. 1 to No. 3450 in SEQ ID NO: 1. A region was found which codes for an amino acid sequence identical to that of the N-terminal of HPGp130 polypeptide. An open reading frame was obtained from HPG3.5k DNA in the same frame as that of HPGp130 polypeptide and it was found that translation starts at nucleotide No. 243 to code for 1069 amino acid residues. There was no termination codon within the region and thus it was assumed that HPG3.5k DNA codes for a portion of HPGp130 polypeptide. A corresponding amino acid sequence is also shown.

### (7) Cloning of HPG4.1k DNA

The above HPG3.5k DNA fragment was labeled with DIG as described above. After the genomic DNA of Haemophilus paragallinarum serotype A strain 221 was cleaved with restriction enzymes XhoI and XbaI, a Southern hybridization was carried out as described in Example 3 (3) using the DIG-labeled HPG3.5k DNA or the DIG-labeled HPG1.2k DNA as a probe. As a result, DNAs of about 5.5 kb, about 4.1 kb and about 1 kb were detected with the DIG-labeled HPG3.5k DNA as a probe. When the DIG-labeled HPG1.2k DNA was used as a probe, DNAs of about 4.1 kb and about 1 kb were detected. Since there are two XhoI sites within the HPG3.5k DNA fragment as shown in Fig. 7, it was considered that the DNA of about 5.5 kb was a fragment corresponding to the 5' site from the first XhoI cleavage site, the DNA of about 4.1 kb was a fragment corresponding to the 3' site from the second XhoI cleavage site and the DNA of about 1 kb was a fragment between these two XhoI sites. Thus, the fragment of about 4.1 kb was separated and recovered on 0.8 % agarose gel electrophoresis.

As shown in Fig. 8, plasmid pSP72 (manufactured by Promega) was digested with XhoI and XbaI and, after dephosphorizing the 5' end, ligated with the above XhoI-XbaI digest (about 4.1 kb) derived from the genome of Haemophilus paragallinarum serotype A strain 221. E.coli strain JM109 cells were transformed with the ligated product. For the obtained E.coli transformants, a colony hybridization was carried out using the DIG-labeled HPG3.5k DNA as a probe to screen positive clones.

The positive clones were cultured on Circle Grow medium containing 50 µg/ml of ampicillin. Plasmids were recovered from the cells by PEG precipitation method. The obtained plasmid (hereinafter referred to as "pSA4.1"), in which the XhoI-XbaI digest fragment (hereinafter referred to as "HPG4.1k DNA") derived from Haemophilus paragallinarum serotype A strain 221 was incorporated, was digested with XhoI and XpnI and then electrophoresed on 0.8 % agarose gel to separate and recover a DNA fragment of about 4.1 kb, which was the above HPG4.1k DNA added with XbaI-KpnI fragment from the plasmid pSP72.

### (8) Expression of HPG4.1k DNA

As described in Example 3 (4), the expression vector pTrcHisC was digested with XhoI and XpnI and, after dephosphorizing the 5' end, ligated with the above XhoI-XpnI digest of about 4.1 kb. E.coli strain JM109 cells were transformed with the ligated product. From the obtained transformants of E.coli, there was obtained E.coli which was transformed with a recombinant plasmid wherein HPG4.1k DNA was ligated in a right direction and expresses an antigen specifically reactive with anti-HPGp130 guinea pig serum.

### (9) Immunogenicity of HPG4.1k-HIS polypeptide

The obtained transformants of E.coli were inoculated to 200 ml of Circle Grow medium containing 50 µg/ml of ampicillin and cultured at 37°C for 3 hours. Thereto was added IPTG (final concentration of 1 mM) and the transformants were cultured at 37°C for additional 3 hours. The cells were harvested from the culture by centrifugation and suspended in 10 ml of PBS. To the suspension was added lysozyme at 100 µg/ml for reaction at 4°C for 1 hour. The suspension was sonicated at 4°C for 10 minutes for bacteriolysis. Intact cells were removed by centrifugation and the obtained supernatant was used as a crude HPG4.1k-HIS polypeptide.

Ten SPF white leghorn chickens of 5 weeks old were immunized by subcutaneously administering at the leg 0.5 ml of an emulsion prepared by thoroughly mixing the crude HPG4.1k-HIS polypeptide solution with the same amount of Freund's complete adjuvant. About three weeks later, the chickens were subcutaneously administered at the leg with 0.5 ml of an emulsion prepared similarly with Freund's incomplete adjuvant. Two weeks later, the chickens were boosted subcutaneously at the leg with an emulsion prepared similarly with Freund's incomplete adjuvant. Seven weeks after the first immunization, the chickens were challenged with Haemophilus paragallinarum serotype A strain 221. As a control, as described in Example 2 (7), one group was immunized with formalin-inactivated HPG serotype A strain 221 and another group was not immunized and both control groups were challenged similarly. The results are shown in Table 4. The group immunized with the crude HPG4.1k-HIS polypeptide showed protection against the onset of the disease in every ten among the tested chickens. The group immunized with the formalin-inactivated cells exhibited protection against the onset of the disease in all the chickens whereas the non-immunization group showed the symptoms in all the chickens.

**Table 4**

| Immunization group | Tested chicken | Protected chicken | Protection rate (%) |
|---|---|---|---|
| Crude HPGp4.1k-HIS | 10 | 10 | 100 |
| Formalin-inactivated strain 221 | 10 | 10 | 100 |
| Non immunization control | 10 | 0 | 0 |

### (10) Nucleotide sequence of HPG4.1k DNA fragment

A nucleotide sequence of a region in HPG4.1k DNA fragment which does not overlap with HPG3.5k DNA fragment, i.e. a region ranging from the HindIII cleavage site to the XbaI cleavage site, was analyzed with a DNA sequencer as described above. As a result, a sequence of 2831 nucleotides was determined. The analyzed nucleotide sequence of HPG4.1k DNA fragment corresponds to the nucleotide sequence of from nucleotides No. 3445 to No. 6275 in SEQ ID NO: 1. No termination codon was found within the region of said DNA fragment. A corresponding amino acid sequence is also shown.

### (11) Cloning of HPG6.7k DNA

After the genomic DNA of Haemophilus paragallinarum serotype A strain 221 was cleaved with XhoI and PstI, a Southern hybridization was carried out as described in Example 3 (3) using the DIG-labeled HPG3.5k DNA or the DIG-labeled HPG1.2k DNA as a probe. As a result, DNAs of about 9.4 kb, about 6.7 kb and about 1 kb were detected with the DIG-labeled HPG3.5k DNA as a probe. When the DIG-labeled HPG1.2k DNA was used as a probe, DNAs of about 6.7 kb and about 1 kb were detected. Since there are two XhoI cleavage sites within the HPG3.5k DNA fragment as described above, it was considered that the DNA of about 9.4 kb was a fragment corresponding to the 5' site from the first XhoI cleavage site, the DNA of about 6.7 kb was a fragment corresponding to the 3' site from the second XhoI cleavage site and the DNA of about 1 kb was a fragment between these two XhoI sites. Thus, the fragment of about 6.7 kb was separated and recovered on 0.8 % agarose gel electrophoresis.

As shown in Fig. 9, plasmid pSP72 was digested with XhoI and PstI and, after dephosphorizing the 5' end, ligated with the above XhoI-PstI digest (about 6.7 kb) derived from the genome of Haemophilus Paragallinarum serotype A strain 221. E.coli strain JM109 cells were transformed with the ligated product. For the obtained E.coli transformants, a colony hybridization was carried out using the DIG-labeled HPG3.5k DNA as a probe to screen positive clones.

The positive clones were cultured on Circle Grow medium containing 50 µg/ml of ampicillin. Plasmids were recovered from the cells by PEG precipitation method. The obtained recombinant plasmid is hereinafter referred to as "pSA6.7". E.coli SA6.7JM transformed with the recombinant plasmid has been deposited by the applicant as FERM BP-6081 at National Institute of Bioscience and Human-Technology Agency of Industrial Science and Technology (1-3, Higashi 1-chome, Tsukuba-shi, Ibaraki-ken) on August 27, 1997.

### (12) Cloning of HPG2.7k DNA

Since the DNA fragment of about 6.7 kb (hereinafter referred to as "HPG6.7k DNA") incorporated in the obtained recombinant plasmid (pSA6.7) encompasses the above HPG4.1k DNA, a fragment of about 2.7 kb (hereinafter referred to as "HPG2.7k DNA") was subcloned which is a subtraction of HPG4.1k DNA from HPG6.7k DNA. pSA6.7 was digested with XbaI and then electrophoresed on 0.8 % agarose gel to separate and recover a DNA fragment of about 2.7 kb which was the above HPG2.7k DNA added with PstI-XbaI fragment from the plasmid pSP72.

Plasmid pSP72 was then digested with XbaI and, after dephosphorizing the 5' end, ligated with the above XbaI digest of about 2.7 kb. E.coli strain JM109 cells were transformed with the ligated product. The obtained E.coli transformants were cultured on Circle Grow medium containing 50 µg/ml of ampicillin. Plasmids were recovered from the cells by PEG precipitation method. The obtained recombinant plasmid is hereinafter referred to as "pSA2.7".

### (13) Nucleotide sequence of HPG2.7k DNA

A nucleotide sequence of HPG2.7k DNA fragment was analyzed with a DNA sequencer as described above. As a result, a sequence of 2661 nucleotides was determined. The nucleotide sequence of HPG2.7k DNA fragment corresponds to the nucleotide sequence of from nucleotides No. 6270 to No. 8930 in SEQ ID NO: 1. A termination codon was found within the region. A corresponding amino acid sequence is also shown.

It was found that the nucleotide sequence of SEQ ID NO: I, consisting of a total of 8930 nucleotides, included an open reading frame starting from nucleotide No. 243 which can code for 2042 amino acid residues. A polypeptide comprising the 2042 amino acid residues is hereinafter referred to as serotype A HMTp210". Homology search with the existing data base (GeneBank and EMBL) revealed no homology with any known nucleotide and amino acid sequences, indicating that the serotype A HMTp210 polypeptide is a novel substance.

The presence of another possible open reading frame in the nucleotide sequence of SEQ ID NO: 1 was also suggested which starts from nucleotide No. 8375 and can code for 185 amino acid residues. No termination codon was found in this sequence. Homology search with the existing data base (GeneBank and EMBL) revealed no homology with any known nucleotide and amino acid sequences, indicating that the polypeptide coded by this open reading frame is also a novel substance.

### Example 4: Search for DNA fragment hybridizable to HPG1.2k DNA from other strains than Haemophilus paragallinarum serotype A strain 221

As described in Example 3 (1), genomic DNAs were prepared from a total of nine strains, i.e. HPG serotype A strains 221, 083, W, Germany and Georgia, HPG serotype B strains Spross and 0222, and HPG serotype C strains Modesto and 53-47. After the prepared genomic DNAs were cleaved with restriction enzyme EcoRI, a Southern hybridization was carried out using the DIG-labeled HPG1.2k DNA as a probe as described in Example 3 (3). As a result, fragments hybridizable with HPG1.2k DNA were detected in every strains although size of each fragment was varied depending on the strains (Fig. 10).

### Example 5: Cloning of gene coding for polypeptide (serotype C HMTp210) from Haemophilus paragallinarum serotype C

### (1) Screening from genomic library

A genomic library of Haemophilus paragallinarum serotype C strain 53-47 was prepared in the same manner as described in Example 3 (1). That is, a genomic DNA of HPG serotype C strain 53-47 digested with restriction enzyme HindIII was ligated to λDASHII (manufactured by STRATAGENE) arm digested with restriction enzyme HindIII using cDNA Rapid Cloning Module-λgt11. Using λ-DNA in vitro packaging module, the ligated product was inserted into λ phage. The obtained solutions of recombinant phage were used as a genomic library.

The above solutions of genomic library were added to a suspension of E.coli strain XL1-Blue MRA (P2) (manufactured by STRATAGENE) about 10⁸ cells in an aqueous solution of 10 mM magnesium sulfate for absorption at 37°C for 15 minutes. Thereto was added LB soft agarose medium (containing tryptone 10 g, yeast extract 5 g, sodium chloride 10 g, ampicillin 50 mg, maltose 4 g and agarose 8 g in 1000 ml, pH 7) for overlay warmed at 45°C. The mixture was overlaid to LB agar medium and incubated at 37°C overnight. To the agar medium where transformed E.coli grown was overlaid Hybond N+ membrane to lift the phage plaques. Using the DIG-labeled serotype A HPG3.5k DNA as a probe, a plaque hybridization was carried out in the conventional manner and positive clones were screened. About 1,000 plaques were immunologically screened as described above to give 37 positive plaques. Ten among the obtained positive plaques were further subjected to second and third screening as in the primary screening.

The recombinant λDASHII phages found positive in the plaque hybridization were added to a suspension of E. coli strain XL1-Blue MRA (manufactured by STRATAGENE) about 10⁸ cells in an aqueous solution of 10 mM magnesium sulfate for absorption at 37°C for 15 minutes. As described in Example 3 (1), the phage DNA was recovered. The obtained phage DNA was digested with HindIII and then electrophoresed on 0.8 % agarose gel to separate and recover DNA fragments derived from Haemophilus paragallinarum serotype C strain 53-47. All the DNA fragments obtained from ten positive phages had a length of about 13.5 kb. A DNA fragment (hereinafter referred to as "HPG-C1 DNA") obtained from the phage of a clone (clone 1) was used in the following test.

### (2) Fragmentation and subcloning of HPG-C1 DNA

Since the HPG-C1 DNA of about 13.5 kb is too large to be subcloned into a plasmid vector, it was cleaved with several restriction enzymes and a suitable amount of the resulting DNA fragments was electrophoresed on 0.8 % agarose gel. As a result, DNA fragments of about 6.9 kb, about 5.6 kb and about 0.9 kb were detected when digested with XbaI.

Plasmid pUC119 was digested with HindIII and XbaI and, after dephosphorizing the 5' end, ligated with the above XbaI digests of HPG-C1 DNA. E.coli strain JM109 cells were transformed with the ligated products. Furthermore, E.coli cells transformed with the recombinant plasmid containing either DNA fragment of about 5.6 kb or about 0.9 kb were cultured and the plasmids were recovered from the cells by PEG precipitation method. The obtained recombinant plasmids (hereinafter referred to as "pU-C2" and "pU-C3", containing either DNA fragment of about 5.6 kb and about 0.9 kb, respectively) was digested with HindIII-XbaI and then electrophoresed on 0.8 % agarose gel to separate and recover DNA fragments of about 5.6 kb and about 0.9 kb (hereinafter referred to as "HPG-C2 DNA" and "HPG-C3 DNA", respectively). E.coli U-C2JM transformed with the recombinant plasmid pU-C2 has been deposited by the applicant as FERM BP-6082 at National Institute of Bioscience and Human-Technology Agency of Industrial Science and Technology (1-3, Higashi 1-chome, Tsukuba-shi, Ibaraki-ken) on August 27, 1997.

Plasmid pUC119 was digested with XbaI and, after dephosphorizing the 5' end, ligated with the above XbaI digests of HPG-C1 DNA. E.coli strain JM109 cells were transformed with the ligated products. Furthermore, E.coli cells transformed with the recombinant plasmid containing DNA fragment of about 6.9 kb were cultured and the plasmid was recovered from the cells by PEG precipitation method. The obtained recombinant plasmid (hereinafter referred to as "pU-C4") was digested with XbaI and then electrophoresed on 0.8 % agarose gel to separate and recover DNA fragment of about 6.9 kb (hereinafter referred to as "HPG-C4 DNA"). E.coli U-C4JM transformed with the recombinant plasmid pU-C4 has been deposited by the applicant as FERM BP-6080 at National Institute of Bioscience and Human-Technology Agency of Industrial Science and Technology (1-3, Higashi 1-chome, Tsukuba-shi, Ibaraki-ken) on August 27, 1997.

Each of the obtained DNA fragments HPG-C2, HPG-C3 and HPG-C4 was spotted on Hybond N+ membrane. Then, a dot hybridization was carried out using as a probe either the above DIG-labeled HPG3.5k DNA or HPG4.1k or HPG2.7k DNA labeled similarly with DIG. When the DIG-labeled HPG3.5k DNA or DIG-labeled HPG4.1k DNA was used as a probe, HPG-C4 DNA was detected. On the other hand, when HPG2.7k DNA was used as a probe, HPG-C2 DNA was detected. From this, it was assumed that HPG-C3, HPG-C4 and HPG-C2 were positioned in this order from the 5' site and HPG-C4 mainly encompasses a region coding for the polypeptide as shown in Fig. 11.

### (3) Nucleotide sequence of HPG-C4 DNA fragment

A nucleotide sequence of HPG-C4 DNA fragment was analyzed with a DNA sequencer as described above. As a result, a sequence of 6871 nucleotides was determined. The nucleotide sequence of HPG-C4 DNA fragment corresponds to the nucleotide sequence of from nucleotides No. 1 to No. 6871 in SEQ ID NO: 5. Based on high homology with the gene coding for serotype A HMTp210, an open reading frame was obtained from HPG-C4 DNA in the same frame as that of the gene coding for serotype A HMTp210 and it was found that translation starts at nucleotide No. 848 to code for 2008 amino acid residues. However, no termination codon was found within the region of said DNA fragment. A corresponding amino acid sequence was also shown.

### (4) Nucleotide sequence of a portion of HPG-C2 DNA fragment

Since no termination codon was found within the region of HPG-C4 DNA fragment, a nucleotide sequence at the 5' site of HPG-C2 DNA fragment, which is at the 3' site of HPG-C4 DNA fragment, was analyzed. As shown in Fig. 11, there are three AccI cleavage sites within HPG-C2 DNA fragment. It was also revealed that a fragment ranging from the cloning site, i.e. XbaI cleavage site, to the first AccI cleavage site is of size about 0.6 Kb as demonstrated in an agarose gel electrophoresis. Thus, a nucleotide sequence of this fragment of about 0.6 Kb was analyzed with a DNA sequencer as described above. As a result, a sequence of 621 nucleotides was determined. The nucleotide sequence of a portion of HPG-C2 DNA fragment corresponds to the nucleotide sequence of from nucleotides No. 6866 to No. 7486 in SEQ ID NO: 5. A termination codon was found within the region of this portion of HPG-C2 DNA fragment. A corresponding amino acid sequence was also shown.

It was found that the nucleotide sequence of SEQ ID NO: 5, consisting of a total of 7486 nucleotides, included an open reading frame starting from nucleotide No. 848 which can code for 2039 amino acid residues. A polypeptide comprising the 2039 amino acid residues is hereinafter referred to as "serotype C HMTp210". Homology search with the existing data base (GeneBank and EMBL) revealed no homology with any known nucleotide and amino acid sequences, indicating that the serotype C HMTp210 polypeptide is a novel substance.

Homology search between the nucleotide sequences coding for the serotype C HMTp210 polypeptide and the serotype A HMTp210 polypeptide revealed about 80 % homology. It was further revealed that the region of about 3.4 kb at the 5' site and the region of about 1.2 kb at the 3' site exhibited extremely high homology whereas the region of about 1.5 kb between these 5' and 3' regions showed low homology. The same was also applicable to the corresponding polypeptides encoded by these genes.

### Example 6: PCR amplification of HMTp210 gene from genomic DNA of HPG serotypes A, B and C cells

As described in Example 3 (1), genomic DNAs were prepared from a total of nine strains, i.e. HPG serotype A strains 221, 083, W, Germany and Georgia, HPG serotype B strains Spross and 0222, and HPG serotype C strains Modesto and 53-47. Based on the nucleotide sequence coding for the Type A HMTp210 polypeptide, there were prepared a synthetic DNA having the nucleotide sequence of SEQ ID NO: 3 as an upstream PCR primer and a synthetic DNA having the nucleotide sequence of SEQ ID NO: 4 as a downstream PCR primer. These primers were designed such that BamHI recognition sequences were added at the 5' site, respectively, and a full length of translation region of the serotype A HMTp210 polypeptide can be amplified. Using these primers, PCR was carried out using the genomic DNAs prepared as mentioned above as a template. PCR was carried out with LA PCR Kit ver. 2 (manufactured by Takara Shuzo K.K.) under the following conditions: after reaction at 94°C for 1 minute, 30 cycles of reactions at 98°C for 40 seconds and at 60°C for 10 minutes, followed by reaction at 72°C for 10 minutes. Analysis of the obtained PCR products on 0.8 % agarose gel electrophoresis confirmed the amplified fragment of about 6.1 Kb in any of these strains (Fig. 12).

### Example 7: Expression of full-length serotypes A and C HMTp210 polypeptides

### (1) Expression of serotype A HMTp210 polypeptide

The PCR product obtained in Example 6 with the genomic DNA from Haemophilus paragallinarum serotype A strain 221 as a template was digested with BamHI. After separation on 0.8 % agarose gel electrophoresis, the amplified fraction of about 6.1 Kb was eluted and recovered with Sephaglas™ BandPrep Kit.

Plasmid pUC119 was digested with BamHI and, after dephosphorizing the 5' end, ligated with the above amplified fragment of about 6.1 kb. E.coli strain JM109 cells were transformed with the ligated product. Furthermore, E.coli cells transformed with the recombinant plasmid containing DNA fragment of about 6.1 kb were cultured and the plasmid was recovered from the cells by PEG precipitation method. The obtained recombinant plasmid (hereinafter referred to as "pU-AP1") was digested with BamHI and then electrophoresed on 0.8 % agarose gel to separate and recover DNA fragment of about 6.1 kb (hereinafter referred to as "HPG-AP1 DNA").

As described in Example 3 (4), the expression vector pTrcHisA (manufactured by Invitrogen) was digested with BamHI and, after dephosphorizing the 5' end, ligated with the above HPG-AP1 DNA. E.coli strain JM109 cells were transformed with the ligated product. From the obtained transformants of E.coli, there was obtained E.coli which was transformed with a recombinant plasmid wherein HPG-AP1 DNA was ligated in a right direction and expressed an antigen specifically reactive with anti-HPGp130 guinea pig serum.

### (2) Expression of serotype C HMTp210 polypeptide

The PCR product obtained in Example 6 with the genomic DNA from Haemophilus paragallinarum serotype C strain 53-47 as a template was digested with BamHI. After separation on 0.8 % agarose gel electrophoresis, the amplified fraction of about 6.1 kb was recovered.

Plasmid pUC119 was digested with BamHI and, after dephosphorizing the 5' end, ligated with the above amplified fragment of about 6.1 kb. E.coli strain JM109 cells were transformed with the ligated product. Furthermore, E.coli cells transformed with the recombinant plasmid containing DNA fragment of about 6.1 kb were cultured and the plasmid was recovered from the cells by PEG precipitation method. The obtained recombinant plasmid (hereinafter referred to as "pU-CP1") was digested with BamHI and then electrophoresed on 0.8 % agarose gel to separate and recover DNA fragment of about 6.1 kb (hereinafter referred to as "HPG-CP1 DNA").

As described in Example 3 (4), the expression vector pTrcHisA (manufactured by Invitrogen) was digested with BamHI and, after dephosphorizing the 5' end, ligated with the above HPG-CP1 DNA. E.coli strain JM109 cells were transformed with the ligated product. From the obtained transformants of E.coli, there was obtained E.coli which was transformed with a recombinant plasmid wherein HPG-CP1 DNA was ligated in a right direction and expressed an antigen specifically reactive with anti-HPGp130 guinea pig serum.

### SEQUENCE LISTING

(1) GENERAL INFORMATION:
   (i) APPLICANT:
      (A) NAME: Juridical Foundation The Chemo-Sero-Therapeutic Research Institute
      (B) STREET: 6-1, Okubo 1-chome
      (C) CITY: Kumamoto-ken
      (D) STATE: Kumamoto-shi
      (E) COUNTRY: Japan
      (F) POSTAL CODE (ZIP): 860
   (ii) TITLE OF INVENTION: NOVEL POLYPEPTIDE FROM HAEHOPHILUS PARAGALLINARUM AND PROCESS FOR PREPARING THE SAME
   (iii) NUMBER OF SEQUENCES: 5
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: EP 97 94 0361.5

SEQ ID NO.: 1
   SEQUENCE LENGTH: 8930
   SEQUENCE TYPE: nucleic acid
   STRANDEDNESS: double
   TOPOLOGY: linear
   MOLECULE TYPE: genomic DNA
   ORIGINAL SOURCE: Haemophilus paragallinarum serotype A strain 221
SEQUENCE DESCRIPTION: 
AAGCTTTTTC GGGCGATTGA AGACGGAATG TTACTTTGGC AAGCGGTTTG AAACCTTTGA 60
ACAGCTTGAA AAAGTGATTC ACGAGTACAT TCATTACTAC AACAATGAGC GTATTCAAGT 120
GAAGCTCAAA GGACTAAGCC CTGTGGAATA CAGAACTCAG TCCTTGAATG AAATTAGAAT 180
ATAGTCTAAC TTTTTGGGGC AGATCAACAC TCATTTTTAA TATTAATATA GGAAAATGAT 240
TT ATG AAT AAA GTT TTT AAA ATT AAA TAT TCT GTT GTA AAA CAA GAA 287
Met Asn Lys Val Phe Lys Ile Lys Tyr Ser Val Val Lys Gln Glu
-70 -65 -60
ATG ATT GTG GTT TCA GAG CTA GCA AAT AAT AAA GAT AAA ACA GCT AGC 335
Met Ile Val Val Ser Glu Leu Ala Asn Asn Lys Asp Lys Thr Ala Ser
-55 -50 -45 -40
 
CAA AAA AAC ACA CAT AAT ACT GCA TTT TTT CAA CCG CTA TTT ACA AAG 383
Gln Lys Asn Thr His Asn Thr Ala Phe Phe Gln Pro Leu Phe Thr Lys
-35 -30 -25
TGT ACA TAT CTT GCT CTT CTC ATT AAT ATC GCA CTA GGA GCA TCA TTA 431
Cys Thr Tyr Leu Ala Leu Leu Ile Asn Ile Ala Leu Gly Ala Ser Leu
-20 -15 -10
TTC CCT CAA TTA GCT AAT GCG AAG TGG TTA GAG GTT TAT AGT AGC TCC 479
Phe Pro Gln Leu Ala Asn Ala Lys Trp Leu Glu Val Tyr Ser Ser Ser
-5 1 5
GTA AAA CTA TCT ACT GTT AGT GCA CAA AGT AAT AGT GTT AAT CTT AAT 527
Val Lys Leu Ser Thr Val Ser Ala Gln Ser Asn Ser Val Asn Leu Asn
10 15 20 25
CCA TCG GGA GCT GAG AGT GTT GGC ACA AAT AGC CCA CAA GGG GTT GCT 575
Pro Ser Gly Ala Glu Ser Val Gly Thr Asn Ser Pro Gln Gly Val Ala
30 35 40
ATT GGC TAT GGT GCA ACC AAC GAT AGA TCT GCA ACA GGA GCT ATT GCT 623
Ile Gly Tyr Gly Ala Thr Asn Asp Arg Ser Ala Thr Gly Ala Ile Ala
45 50 55
CTT GGG GTT GGG GTA AAA AAT GAA ACT TTA GCG AAA GAC TCT ATT GCC 671
Leu Gly Val Gly Val Lys Asn Glu Thr Leu Ala Lys Asp Ser Ile Ala
60 65 70
ATT GGT TAT GGG GCA AAA AAT GAA AGC ACA GCA CCA AGT TCT GTG ACT 719
Ile Gly Tyr Gly Ala Lys Asn Glu Ser Thr Ala Pro Ser Ser Val Thr
75 80 85
ATT GGA AAA CAG GCG ATT AAC CGT TTT GAA AAA TCT ATT GTG ATG GGT 767
Ile Gly Lys Gln Ala Ile Asn Arg Phe Glu Lys Ser Ile Val Met Gly
90 95 100 105
CTT AAT GCT TAT ACA CAA TTA GAT CCC CGT GGA ACT AGT AAA GAA ACC 815
Leu Asn Ala Tyr Thr Gln Leu Asp Pro Arg Gly Thr Ser Lys Glu Thr
110 115 120
CGT CAA GGT TCT GTA GTG ATT GGG GAA AAT GCG AAA AGT GCT GGG AAT 863
Arg Gln Gly Ser Val Val Ile Gly Glu Asn Ala Lys Ser Ala Gly Asn
125 130 135
 
CAA TCT GTT TCT TTA GGG CAA AAT TCG TGG TCA AAA ACG AAT TCT ATT 911
Gln Ser Val Ser Leu Gly Gln Asn Ser Trp Ser Lys Thr Asn Ser Ile
140 145 150
TCT ATT GGG GCA GGA ACC TTT GCG GAA GGA AAA TCA AGC ATT GCT ATA 959
Ser Ile Gly Ala Gly Thr Phe Ala Glu Gly Lys Ser Ser Ile Ala Ile
155 160 165
GGG ACT GAT AAA ATA TCA GGG ACT AAG TAT AAT GAC AAA TTG CCT GCT 1007
Gly Thr Asp Lys Ile Ser Gly Thr Lys Tyr Asn Asp Lys Leu Pro Ala
170 175 180 185
ACT GCT TGG AAT GGA ACA GGC ACT GTT CCG AAA AAC TCC ATT TGG GAT 1055
Thr Ala Trp Asn Gly Thr Gly Thr Val Pro Lys Asn Ser Ile Trp Asp
190 195 200
ATA TTT TCT GAG TTA TAT ATG GGG AAA CAG ACT AAC GGC AGA GAT TAT 1103
Ile Phe Ser Glu Leu Tyr Met Gly Lys Gln Thr Asn Gly Arg Asp Tyr
205 210 215
GAT ACA ACT ACT CGA GAC CCT AAT AAA CCG GAG GCA TTT TAT AAA TTT 1151
Asp Thr Thr Thr Arg Asp Pro Asn Lys Pro Glu Ala Phe Tyr Lys Phe
220 225 230
AGC GAT TTT AAA GGA AAA TAT GTC AAT ACC CCA ACT GCT TCA CCT ACT 1199
Ser Asp Phe Lys Gly Lys Tyr Val Asn Thr Pro Thr Ala Ser Pro Thr
235 240 245
TAT GCA GGG AAA TTA GGG GCA ATT GCT CTA GGT TCC CGC ACC ATT GCC 1247
Tyr Ala Gly Lys Leu Gly Ala Ile Ala Leu Gly Ser Arg Thr Ile Ala
250 255 260 265
GCG GGG GAA ATG TCC ACC GCA GTG GGT TCG TTA GCC TTT GCA TTG GCA 1295
Ala Gly Glu Met Ser Thr Ala Val Gly Ser Leu Ala Phe Ala Leu Ala
270 275 280
GAT AGA TCC ACC GCA ATG GGG TTA CGT TCT TTT GTT GCT AAA GAC GCC 1343
Asp Arg Ser Thr Ala Met Gly Leu Arg Ser Phe Val Ala Lys Asp Ala
285 290 295
GTA GGT GGA ACG GCG ATC GGG GAA GAA TCT CGA ACC TTT GCT AAA GAT 1391
Val Gly Gly Thr Ala Ile Gly Glu Glu Ser Arg Thr Phe Ala Lys Asp
300 305 310
 
TCC GTT GCC ATT GGT AAT AAA ACT GAA GCC TCA AAT GCT GGC TCA ATG 1439
Ser Val Ala Ile Gly Asn Lys Thr Glu Ala Ser Asn Ala Gly Ser Met
315 320 325
GCT TAT GGT TAT AAG GCG AAA GCA GTA GGT GCG GGA GCA ATC GCA ATT 1487
Ala Tyr Gly Tyr Lys Ala Lys Ala Val Gly Ala Gly Ala Ile Ala Ile
330 335 340 345
GGG ACA GAA GTC GCA GCA GGG GCT AAA TTT AAT AGC CAT CAA ACA GGA 1535
Gly Thr Glu Val Ala Ala Gly Ala Lys Phe Asn Ser His Gln Thr Gly
350 355 360
AAT TTA CTA CAG GAT AAT AAT GCT TAT GCT ACC TTA AAA AAT GCC GAT 1583
Asn Leu Leu Gln Asp Asn Asn Ala Tyr Ala Thr Leu Lys Asn Ala Asp
365 370 375
AAA TCA GAT GAT ACT AAA ACC GGA AAT GCG ATT ACT GTA TTT ACC CAG 1631
Lys Ser Asp Asp Thr Lys Thr Gly Asn Ala Ile Thr Val Phe Thr Gln
380 385 390
TCT TTT GAT AAT ATG CTT ACT AAT GGA TTA CCG CTG GTA AGT GAA AAC 1679
Ser Phe Asp Asn Met Leu Thr Asn Gly Leu Pro Leu Val Ser Glu Asn
395 400 405
GAA ACC TAT TTA ACG ACC TCA GCG GGA GCA ATT AAA AAA ACT GCA ACA 1727
Glu Thr Tyr Leu Thr Thr Ser Ala Gly Ala Ile Lys Lys Thr Ala Thr
410 415 420 425
ACA GAC AGC AGT GCG GGG GGA GGT AAA AAT GCC ATT GCA ATT GGT AGT 1775
Thr Asp Ser Ser Ala Gly Gly Gly Lys Asn Ala Ile Ala Ile Gly Ser
430 435 440
AAA ACC TTT GCC TCT AAA GCA AAT TCT GTG GCA TTA GGG AGC TAT GCC 1823
Lys Thr Phe Ala Ser Lys Ala Asn Ser Val Ala Leu Gly Ser Tyr Ala
445 450 455
TTA GCC GAT GCC CAA AAT GCC TTT GCA CTA GGT TCT TAT TCT TTT GTG 1871
Leu Ala Asp Ala Gln Asn Ala Phe Ala Leu Gly Ser Tyr Ser Phe Val
460 465 470
GAA TCT TCA GCA ACA AAT ACA ATC ACA ATT GGT GTG GGA AGT TAT GCC 1919
Glu Ser Ser Ala Thr Asn Thr Ile Thr Ile Gly Val Gly Ser Tyr Ala
475 480 485
 
AAA GGG AAA AAC AGT TTC TTA GGG GGG ACT TGG GCA TCA ACC CTT TCA 1967
Lys Gly Lys Asn Ser Phe Leu Gly Gly Thr Trp Ala Ser Thr Leu Ser
490 495 500 505
GAT CGG ACA GTT GTG CTA GGG AAT TCC ACT TCA ATT AGC TCA GGT TCT 2015
Asp Arg Thr Val Val Leu Gly Asn Ser Thr Ser Ile Ser Ser Gly Ser
510 515 520
CAG AAT GCA TTA GCA ATC GGG GTG AAT GTC TTT ATT GGT AAT GAT AGT 2063
Gln Asn Ala Leu Ala Ile Gly Val Asn Val Phe Ile Gly Asn Asp Ser
525 530 535
GCT TCT TCA TTG GCA TTA GGT ATG GGT TCT ACT ATT GCG AAA AGT GCC 2111
Ala Ser Ser Leu Ala Leu Gly Met Gly Ser Thr Ile Ala Lys Ser Ala
540 545 550
AAA TCC CCT GAC AGC TTA GCC ATT GGT AAA GAG GCA CGA ATT GAC GCT 2159
Lys Ser Pro Asp Ser Leu Ala Ile Gly Lys Glu Ala Arg Ile Asp Ala
555 560 565
AAA GAT ACA GAT AAT GGT ACT TTG TAT CAG CCT CAA GTT TAT GAT GAA 2207
Lys Asp Thr Asp Asn Gly Thr Leu Tyr Gln Pro Gln Val Tyr Asp Glu
570 575 580 585
ACT ACT CGA GCC TTT AGA AAC TTT AAT GAA AGT AGC GAT TAT ATG CGT 2255
Thr Thr Arg Ala Phe Arg Asn Phe Asn Glu Ser Ser Asp Tyr Met Arg
590 595 600
CAA GCA ATG GCA TTA GGT TTT AAT GCT AAA GTT TCG CGT GGG GTG GGC 2303
Gln Ala Met Ala Leu Gly Phe Asn Ala Lys Val Ser Arg Gly Val Gly
605 610 615
AAA ATG GAA ACG GGG ATT AAC TCG ATG GCG ATT GGT GCT TAT GCT CAA 2351
Lys Met Glu Thr Gly Ile Asn Ser Met Ala Ile Gly Ala Tyr Ala Gln
620 625 630
GCA ACT TTG CAA AAT TCC ACC GCA CTT GGG GTA GGC TCT AAA ACA GAT 2399
Ala Thr Leu Gln Asn Ser Thr Ala Leu Gly Val Gly Ser Lys Thr Asp
635 640 645
TAC ACT TGG GAA CAG TTA GAA ACC GAT CCT TGG GTA TCT GAA GGG GCA 2447
Tyr Thr Trp Glu Gln Leu Glu Thr Asp Pro Trp Val Ser Glu Gly Ala
650 655 660 665
 
ATC AGT ATC CCA ACT TCA GGT AAA ACT GGG GTT ATC TCT GTG GGT TCA 2495
Ile Ser Ile Pro Thr Ser Gly Lys Thr Gly Val Ile Ser Val Gly Ser
670 675 680
AAA GGT TCA GAA CGT CGT ATT GTG AAT CTT GCT TCG GGT TCT TCT GAT 2543
Lys Gly Ser Glu Arg Arg Ile Val Asn Leu Ala Ser Gly Ser Ser Asp
685 690 695
ACT GAT GCC GTG AAT GTT GCT CAG TTA AAA ACC GTT GAA GAA CGT TTC 2591
Thr Asp Ala Val Asn Val Ala Gln Leu Lys Thr Val Glu Glu Arg Phe
700 705 710
CTA TCT GAA ATT AAT TTA TTA CAA AAT GGC GGT GGG GTG AAA TAT CTC 2639
Leu Ser Glu Ile Asn Leu Leu Gln Asn Gly Gly Gly Val Lys Tyr Leu
715 720 725
TCT GTT GAA AAA ACG AAT ATC AAT GGA CAA TCG GGG AGA GTG GCT AGC 2687
Ser Val Glu Lys Thr Asn Ile Asn Gly Gln Ser Gly Arg Val Ala Ser
730 735 740 745
CAA ATT CGT AAA GGG GAA AAT TAT GAG CGA TAT GTG AAA TTA AAA ACA 2735
Gln Ile Arg Lys Gly Glu Asn Tyr Glu Arg Tyr Val Lys Leu Lys Thr
750 755 760
CAA TTG CTC TAT TTA GAT GCA CGA GGA AAA TTA AAT GGA GAG AAG TTT 2783
Gln Leu Leu Tyr Leu Asp Ala Arg Gly Lys Leu Asn Gly Glu Lys Phe
765 770 775
GAT CAA AAT TCA TTA AAC AAA ATT CGT GCG GTA GTG CAA GAA CTT GAA 2831
Asp Gln Asn Ser Leu Asn Lys Ile Arg Ala Val Val Gln Glu Leu Glu
780 785 790
GCG GAA TAT AGT GGC GAG TTA AAA ACA ACC GCG TCA GCT CTC AAT CAG 2879
Ala Glu Tyr Ser Gly Glu Leu Lys Thr Thr Ala Ser Ala Leu Asn Gln
795 800 805
GTT GCA ACA CAA TTA GAG CAA GAA GTA ACC ACA AAT AAC TTC GAC AAA 2927
Val Ala Thr Gln Leu Glu Gln Glu Val Thr Thr Asn Asn Phe Asp Lys
810 815 820 825
TTT AAT CAA TAT AAA ACG CAG ATT GAG AAT GCA AGC AAT GCG GAT TCA 2975
Phe Asn Gln Tyr Lys Thr Gln Ile Glu Asn Ala Ser Asn Ala Asp Ser
830 835 840
 
GCA AGA AAT GTA GGC GGC TTA ACC CCT CAA GCA ATT GCA CAG TTA AAA 3023
Ala Arg Asn Val Gly Gly Leu Thr Pro Gln Ala Ile Ala Gln Leu Lys
845 850 855
GCC AAT AAT AAC TAT CTT AAT GAT GGT GCA AAA GGG CAA GAC AGT ATT 3071
Ala Asn Asn Asn Tyr Leu Asn Asp Gly Ala Lys Gly Gln Asp Ser Ile
860 865 870
GCA TTT GGC TGG CAG GCA AAA ACC TCA GGA GCT AAT AAT GGA TTA GCA 3119
Ala Phe Gly Trp Gln Ala Lys Thr Ser Gly Ala Asn Asn Gly Leu Ala
875 880 885
GGG AAA CAA GCC ATT GCG ATT GGT TTC CAA GCG AAT TCT TCC GCT GAA 3167
Gly Lys Gln Ala Ile Ala Ile Gly Phe Gln Ala Asn Ser Ser Ala Glu
890 895 900 905
AAT GCC ATT TCA ATC GGC ACG AAT TCG GAT ACC TCA ATG ACA GGG GCA 3215
Asn Ala Ile Ser Ile Gly Thr Asn Ser Asp Thr Ser Met Thr Gly Ala
910 915 920
GTG GCG ATT GGT AAA GGT GCA ACG GTT ACT GCG GGT GGA AAA CCT TCC 3263
Val Ala Ile Gly Lys Gly Ala Thr Val Thr Ala Gly Gly Lys Pro Ser
925 930 935
ATT GCA TTG GGG CAA GAT TCG ACG GTT GCC AAT TCC GCA ATT AGC CGT 3311
Ile Ala Leu Gly Gln Asp Ser Thr Val Ala Asn Ser Ala Ile Ser Arg
940 945 950
ACA AGT TCA CCG ATG ATA AAT GGT TTA ATA TTC AAT AAT TTT GCA GGT 3359
Thr Ser Ser Pro Met Ile Asn Gly Leu Ile Phe Asn Asn Phe Ala Gly
955 960 965
TCC CCT GAA ACA CTC GGT GTG TTA AGT ATC GGA ACG GCT GGG AGA GAG 3407
Ser Pro Glu Thr Leu Gly Val Leu Ser Ile Gly Thr Ala Gly Arg Glu
970 975 980 985
CGT AAA ATT GTT AAT GTT GCA GCA GGC GAT GTT TCG CAA GCT TCT ACT 3455
Arg Lys Ile Val Asn Val Ala Ala Gly Asp Val Ser Gln Ala Ser Thr
990 995 1000
GAA GCC ATT AAC GGC TCA CAG CTT TAT GCA ACG AAC TTT ATG TTG AGC 3503
Glu Ala Ile Asn Gly Ser Gln Leu Tyr Ala Thr Asn Phe Met Leu Ser
1005 1010 1015
 
AAA GTG GCT CAA TCT GTT AAG AGC AAC TTT GGT GGC•AAT GTA-AAT CTT 3551

Lys Val Ala Gln Ser Val Lys Ser Asn Phe Gly Gly Asn Val Asn Leu

1020 1025 1030

GGC ACT GAT GGC ACA ATT ACA TTT ACA AAT ATT GGC GGC ACA GGG CAA 3599

Gly Thr Asp Gly Thr Ile Thr Phe Thr Asn Ile Gly Gly Thr Gly Gln

1035 1040 1045

GCT ACA ATC CAC GAT GCG ATT AAT AAT GTT CTC ACT AAA GGG ATC TAC 3647

Ala Thr Ile His Asp Ala Ile Asn Asn Val Leu Thr Lys Gly Ile Tyr

1050 1055 1060 1065

CTT AAA GCG GAT CAG AAT GAT CCA ACA GGA AAT CAA GGT CAG AAA GTG 3695

Leu Lys Ala Asp Gln Asn Asp Pro Thr Gly Asn Gln Gly Gln Lys Val

1070 1075 1080

GAA CTT GGT AAT GCA ATA ACG CTT TCG GCA ACA AAT CAA TGG GCG AAT 3743

Glu Leu Gly Asn Ala Ile Thr Leu Ser Ala Thr Asn Gln Trp Ala Asn

1085 1090 1095

AAC GGC GTA AAT TAT AAA ACG AAC AAT TTA ACC ACT TAT AAT TCA CAA 3791

Asn Gly Val Asn Tyr Lys Thr Asn Asn Leu Thr Thr Tyr Asn Ser Gln

1100 1105 1110

AAT GGC ACG ATT TTA TTT GGA ATG CGT GAA GAT CCA AGT GTA AAA CAA 3839

Asn Gly Thr Ile Leu Phe Gly Met Arg Glu Asp Pro Ser Val Lys Gln

1115 1120 1125

ATT ACA GCG GGA ACC TAT AAT ACA ACG GGT GAT GCG AAC AAT AAA AAT 3887

Ile Thr Ala Gly Thr Tyr Asn Thr Thr Gly Asp Ala Asn Asn Lys Asn

1130 1135 1140 1145

CAA CTA AAT AAT ACA CTT CAA CAA ACC ACG CTT GAA GCA ACT GGG ATC 3935

Gln Leu Asn Asn Thr Leu Gln Gln Thr Thr Leu Glu Ala Thr Gly Ile

1150 1155 1160

ACC AGT AGC GTA GGT TCA ACT AAC TAC GCT GGC TTT AGC TTA GGG GCA 3983

Thr Ser Ser Val Gly Ser Thr Asn Tyr Ala Gly Phe Ser Leu Gly Ala

1165 1170 1175

GAC AGC GTC ACC TTC TCG AAA GGT GGA GCT GGC ACG GTG AAA CTT TCT 4031

Asp Ser Val Thr Phe Ser Lys Gly Gly Ala Gly Thr Val Lys Leu Ser

1180 1185 1190
 
GGC GTA AGC GAT GCC ACA GCC GAC ACC GAC GCT GCC ACT CTT AAA CAA 4079
Gly Val Ser Asp Ala Thr Ala Asp Thr Asp Ala Ala Thr Leu Lys Gln
1195 1200 1205
GTG AAA GAA TAC CGC ACA ACA TTA GTG GGT GAT AAT GAC ATC ACC GCA 4127
Val Lys Glu Tyr Arg Thr Thr Leu Val Gly Asp Asn Asp Ile Thr Ala
1210 1215 1220 1225
GCA GAT CGT AGT GGC GGC ACA AGC AAT GGC ATT ACC TAC AAC TTA AGC 4175
Ala Asp Arg Ser Gly Gly Thr Ser Asn Gly Ile Thr Tyr Asn Leu Ser
1230 1235 1240
CTT AAT AAA GGT ACG GTT TCG GCA ACA GAA GAA AAA GTG GTG TCA GGG 4223
Leu Asn Lys Gly Thr Val Ser Ala Thr Glu Glu Lys Val Val Ser Gly
1245 1250 1255
AAA ACT GTC TAT GAA GCC ATT AGA AAT GCC ATC ACA GGC AAC ATC TTC 4271
Lys Thr Val Tyr Glu Ala Ile Arg Asn Ala Ile Thr Gly Asn Ile Phe
1260 1265 1270
ACA ATT GGC TTA GAC GAT ACC ACC TTG AAC AAA ATC AAC AAT CCC GCG 4319
Thr Ile Gly Leu Asp Asp Thr Thr Leu Asn Lys Ile Asn Asn Pro Ala
1275 1280 1285
GAT CAA GAT CTT TCA AAC CTC AGT GAA AGT GGC AAA AAT GCC ATT ACG 4367
Asp Gln Asp Leu Ser Asn Leu Ser Glu Ser Gly Lys Asn Ala Ile Thr
1290 1295 1300 1305
GGC TTA GTG GAT GTG GTG AAA AAA ACA AAT TCA CCG ATC ACA GTT GAG 4415
Gly Leu Val Asp Val Val Lys Lys Thr Asn Ser Pro Ile Thr Val Glu
1310 1315 1320
CCT TCT ACC GAT AGC AAC AAG AAA AAA ACC TTC ACT GTA GGC GTG GAT 4463
Pro Ser Thr Asp Ser Asn Lys Lys Lys Thr Phe Thr Val Gly Val Asp
1325 1330 1335
TTC ACC GAT ACC ATT ACG GAA GGT GAC GCA ACG GAT GAT AAA AAA CTG 4511
Phe Thr Asp Thr Ile Thr Glu Gly Asp Ala Thr Asp Asp Lys Lys Leu
1340 1345 1350
ACG ACT TCA AAA TCC GTT GAA AGC TAT GTC ACA AAC AAA CTC GCG AAC 4559
Thr Thr Ser Lys Ser Val Glu Ser Tyr Val Thr Asn Lys Leu Ala Asn
1355 1360 1365
 
TTC TCT ACA GAT ATT TTG TTA TCG GAT GGG CGT TCT GGT AAC GCA ACA 4607
Phe Ser Thr Asp Ile Leu Leu Ser Asp Gly Arg Ser Gly Asn Ala Thr
1370 1375 1380 1385
ACG GCA AAT GAT GGG GTG GGT AAA CGT CGT TTG TCT GAT GGC TTT ACG 4655
Thr Ala Asn Asp Gly Val Gly Lys Arg Arg Leu Ser Asp Gly Phe Thr
1390 1395 1400
ATC AAA TCT GAA AAC TTT ACG CTA GGT TCA AAA CAA TAT AAT GGC TCT 4703
Ile Lys Ser Glu Asn Phe Thr Leu Gly Ser Lys Gln Tyr Asn Gly Ser
1405 1410 1415
GAT AGC TTA GGG GTA ATG TAT GAC GAT CAA AAT GGG GTC TTT AAA TTA 4751
Asp Ser Leu Gly Val Met Tyr Asp Asp Gln Asn Gly Val Phe Lys Leu
1420 1425 1430
AGC CTA AAT ATG ACC GCA CTT ACC ACT TCA TTG GCT AAT ACT TTC GCG 4799
Ser Leu Asn Met Thr Ala Leu Thr Thr Ser Leu Ala Asn Thr Phe Ala
1435 1440 1445
AAG TTG GAT GCC TCT AAC CTT ACT GAT GAT AGC AAT AAA GAG AAA TGG 4847
Lys Leu Asp Ala Ser Asn Leu Thr Asp Asp Ser Asn Lys Glu Lys Trp
1450 1455 1460 1465
CGT ACT GCG TTG AAT GTG TAT TCA AAA ACA GAA GTA GAT GCA GAA ATT 4895
Arg Thr Ala Leu Asn Val Tyr Ser Lys Thr Glu Val Asp Ala Glu Ile
1470 1475 1480
CAA AAA TCC AAG GTA ACA CTC ACA CCA GAT TCG GGT TTG ATC TTT GCG 4943
Gln Lys Ser Lys Val Thr Leu Thr Pro Asp Ser Gly Leu Ile Phe Ala
1485 1490 1495
ACC AAA CAA GCT GGG AGT GGT AAT AAC GCA GGT ATT GAT GCT GGG AAT 4991
Thr Lys Gln Ala Gly Ser Gly Asn Asn Ala Gly Ile Asp Ala Gly Asn
1500 1505 1510
AAG AAA ATT AGT AAT GTC GCC GAT GGG GAT ATT TCT CCA ACC AGT GGT 5039
Lys Lys Ile Ser Asn Val Ala Asp Gly Asp Ile Ser Pro Thr Ser Gly
1515 1520 1525
GAT GTA GTG ACA GGT CGT CAG CTC TAC GCC TTA ATG CAG AAA GGT ATT 5087
Asp Val Val Thr Gly Arg Gln Leu Tyr Ala Leu Met Gln Lys Gly Ile
1530 1535 1540 1545
 
CGC GTG TAT GGT GAT GAA GTT AGT CCA ACG AAG ACT CAA ACA ACA GCA 5135
Arg Val Tyr Gly Asp Glu Val Ser Pro Thr Lys Thr Gln Thr Thr Ala
1550 1555 1560
CCT ACA AAT GCA AAC CCA ACT GCG ACG ACA GCA CCT ACA GCA TCT AGC 5183
Pro Thr Asn Ala Asn Pro Thr Ala Thr Thr Ala Pro Thr Ala Ser Ser
1565 1570 1575
ACT CAA GGT TGG GCG ACA ACG GCG AAT ACG GCG GGT GGT GTA GCA CCA 5231
Thr Gln Gly Trp Ala Thr Thr Ala Asn Thr Ala Gly Gly Val Ala Pro
1580 1585 1590
GCA GGT AAT GTA GCA ACG GGG GAT ATT GCG CCG ACA CAG CCA ACA TTG 5279
Ala Gly Asn Val Ala Thr Gly Asp Ile Ala Pro Thr Gln Pro Thr Leu
1595 1600 1605
CCA GAG ATG AAT ACG GCA TTG GTT GAT GAT CAC TTG GCT GTG CCG TTA 5327
Pro Glu Met Asn Thr Ala Leu Val Asp Asp His Leu Ala Val Pro Leu
1610 1615 1620 1625
GGT GGA AGC CTC AAG ATT CAC GGA GAT CAT AAT GTG AAA ACA ACG ATT 5375
Gly Gly Ser Leu Lys Ile His Gly Asp His Asn Val Lys Thr Thr Ile
1630 1635 1640
TCT GCG GAT AAT CAA GTG GGG ATT TCA TTA CAG CCA AAT ATT TCT ATT 5423
Ser Ala Asp Asn Gln Val Gly Ile Ser Leu Gln Pro Asn Ile Ser Ile
1645 1650 1655
GAG AAT AAC TTG GTA ATT GGT TCA AAT GAT CCT GAG AAG GCA AAA TTA 5471
Glu Asn Asn Leu Val Ile Gly Ser Asn Asp Pro Glu Lys Ala Lys Leu
1660 1665 1670
GCC GCA CAA GAA GGT AAT GCT TTG GTT ATC ACT AAC AAA GAT GAC GGG 5519
Ala Ala Gln Glu Gly Asn Ala Leu Val Ile Thr Asn Lys Asp Asp Gly
1675 1680 1685
AAT GCG GCG ATG GTC TTT AAT AAC GAG AAA AAT ATG CTT GTT CTC AGT 5567
Asn Ala Ala Met Val Phe Asn Asn Glu Lys Asn Met Leu Val Leu Ser
1690 1695 1700 1705
GAT AAA GAG GCG AAA CCA AGA GTG CTT CTT GAT GGA CAA AAT GGG GCA 5615
Asp Lys Glu Ala Lys Pro Arg Val Leu Leu Asp Gly Gln Asn Gly Ala
1710 1715 1720
 
TTA ACT TTA GTC GGC AAT GAT GAT TCT CAA GTC ACC CTT TCC TCT AAG 5663
Leu Thr Leu Val Gly Asn Asp Asp Ser Gln Val Thr Leu Ser Ser Lys
1725 1730 1735
AAA GGT AAA GAT ATT GAT GGA AAT GAT TTG AGC CGT CTC TCT GTG ACG 5711
Lys Gly Lys Asp Ile Asp Gly Asn Asp Leu Ser Arg Leu Ser Val Thr
1740 1745 1750
ACT GAA AGA ACA AAT GCT GAT GGG CAA CTT GAA AAA GTG GAA ACC TCA 5759
Thr Glu Arg Thr Asn Ala Asp Gly Gln Leu Glu Lys Val Glu Thr Ser
1755 1760 1765
TTT GCT ACA ATG GAT GAT GGC TTG AAG TTC AAA GCC GAC GGG GAT AAA 5807
Phe Ala Thr Met Asp Asp Gly Leu Lys Phe Lys Ala Asp Gly Asp Lys
1770 1775 1780 1785
GTG ATT AAT AAG AAA CTT AAT GAA ACC GTT GAA ATT GTT GGT GAT GAG 5855
Val Ile Asn Lys Lys Leu Asn Glu Thr Val Glu Ile Val Gly Asp Glu
1790 1795 1800
AAT GTG ACA ACA TCT ATT ACT GAT GAT AAT AAG GTG AAA GTT TCA CTG 5903
Asn Val Thr Thr Ser Ile Thr Asp Asp Asn Lys Val Lys Val Ser Leu
1805 1810 1815
AAT AAG AAA ATC GCG ATT GAT GAG GTT AAG ATT CCA AAT ACA GAT CCT 5951
Asn Lys Lys Ile Ala Ile Asp Glu Val Lys Ile Pro Asn Thr Asp Pro
1820 1825 1830
GAT GCT CAA AAG GGA GAT AGC ATT GTA ATC AAC AAT GGT GGA ATC CAC 5999
Asp Ala Gln Lys Gly Asp Ser Ile Val Ile Asn Asn Gly Gly Ile His
1835 1840 1845
GCA GGT AAT AAA GTG ATT ACT GGC GTT AAA GCG AGT GAT GAC CCA ACC 6047
Ala Gly Asn Lys Val Ile Thr Gly Val Lys Ala Ser Asp Asp Pro Thr
1850 1855 1860 1865
AGT GCA GTG AAT CGA GGT CAA TTA AAT ACT GTG ATT GAT AAT GTT CAA 6095
Ser Ala Val Asn Arg Gly Gln Leu Asn Thr Val Ile Asp Asn Val Gln
1870 1875 1880
AAT AAT TTC AAT CAA GTT AAT CAA CGT ATT GGC GAT TTA ACA CGG GAG 6143
Asn Asn Phe Asn Gln Val Asn Gln Arg Ile Gly Asp Leu Thr Arg Glu
1885 1890 1895
 
TCG CGT GCA GGT ATT GCA GGT GCA ATG GCG ACG GCA AGC CTA CAA AAT 6191
Ser Arg Ala Gly Ile Ala Gly Ala Met Ala Thr Ala Ser Leu Gln Asn
1900 1905 1910
GTT GCT TTA CCA GGG AAA ACA ACG ATT TCC GTA GGT ACA GCA ACG TTC 6239
Val Ala Leu Pro Gly Lys Thr Thr Ile Ser Val Gly Thr Ala Thr Phe
1915 1920 1925
AAA GGG GAG AAT GCT GTT GCA ATA GGG ATG TCT AGA CTC TCT GAT AAT 6287
Lys Gly Glu Asn Ala Val Ala Ile Gly Met Ser Arg Leu Ser Asp Asn
1930 1935 1940 1945
GGA AAA GTA GGT ATC CGT TTA TCT GGT ATG AGT ACG AGT AAC GGA GAT 6335
Gly Lys Val Gly Ile Arg Leu Ser Gly Met Ser Thr Ser Asn Gly Asp
1950 1955 1960
AAA GGG GCA GCA ATG AGT GTT GGA TTT AGC TTT TAGCCTTAAT CCATAAATAA 6388
Lys Gly Ala Ala Met Ser Val Gly Phe Ser Phe
1965 1970
GCAAAAAGCG AATCACCTTT GATTCGCTTT TTTTATCAGA TTATGTGCCG TAAAACTCCG 6448
TCCTTCAGGG CGGAGATATA AGGCACAAAC GGCGTAAGCC GTTTCAAACC TAACTAATCA 6508
GGTGTTTGTT GTTGCTCAAT GTATTGGCGA ATAATGGAAA TTGGAGCGCC ACCACAACTC 6568
CCTGCAAAAT AAGACGGAGA CCAAAGCTGA TTACCCCAAA GTTTTTTGCG GATGTTCGAG 6628
TAGTTTTTCT TCCTAATCAT TCGGCTTGAT ACACCTTTTA AACTGTTTAC AAGTGTAGAT 6688
ACAGCCACTT TCGGTGGATA TTCCACAAGT AAATGAACAT GATCGTCTTC ACCGTCAAAT 6748
TCAACTAATT TTGCTTTAAA ATCATTGCAG ACGCTTTCAA AAATCAATTT GAGTTCGTCT 6808
AAAATAGCTT TCGTAAAAAC ATCACGGCGA TATTTTGTTA CAAAGACTAA GTGAACATGC 6868
ATATTAAAAA CACAATGTCT ACCGTGCCTA ATTTCTGTTT CTTTTTGCAT AGACCAAGTG 6928
TAAAATGTTG AAAACTTACA TTCTAAACCT TGTCAATGCA ACTACGCAAA GCCTTTAAGT 6988
TCGAGATAAT GCCGAATGGC GAACAAACCC GTAAAATCAA GCAATTTTGC GGTTGTTCTC 7048
GTTTTGTGTT CAATCGGGCA TTGGCTTGGC AAAATGAACA ATACGGGCAA GATAACAGCT 7108
TTAAGTTCAG TTACACTAAA ATCGCCAACT TGCTTCCACA ATGGAAAAAA GAATTAGTTT 7168
GGCTAAAAGA ATGCCATTCT CAAGTGCTTC AACAGTCGCT AAAAGATCTT GAGAGTGCGT 7228
TCAAAAATTT CTTTCAGAAA CGTGCCGACT TTCCAAAATT CAAGAAAAAA GGCGTGAAAG 7288
AGAGCTTTCG TTTTCCGCAA GGTTGCAAAT TAGAACAGGA AAATGACCGC TTATTTTTGC 7348
CAAAAATCGG CTGGATTCGC TATCGCAACA GCCGAGATAT CGTTGGTGAA ATCAAAAATG 7408
TTACCGTCAG CCAAAAGTGC GGTCACTATT TTGTCAGTAT TCAAACTGAA TTTGAGTACG 7468
 
AAATCCCGAC ACATAAAGGC GGTGAAATCG GTATTGATAT GGGCGTTGCA•CGTTTTGCA[?] 7528

CATTGTCAAA TGGTGAATAT TTTGAACCGG TTAACGCCTT TAAAACTTAC AAAGGAAAAT 7588

TGGCTAAACT GCAAAAGAGG CTTAAAAATA AAGTAAAATT TAGCCAAAAT TGGCAGAAAT 7648

TAAAGGCGAA AATCGCCAAA CTGCATCATA AAATTGCTAA TTGTCGCAAA GACTTCTTGC 7708

ATCAGACTTC AAGCAAAATC AGCAAAAACC ACGCCATGAT CTATATTGAA GATTTGCAGG 7768

TGTCAAATAT GTCAAAATCA GCCAAAGGTA CGGCGGAAAC ACCAGGCAAA AATGTTGCTG 7828

CAAAATCAGG GTTGAACCAA GCGATATTAG ATCAATCTTG GTTTGAGTTT CGCCGTCAGT 7888

TGGACTACAA AACGCAATGG CAAGGTGGAT TTTTAGTGGC AGTGCCAGCG CAAAATACCA 7948

GTCGAACTTG CCCTTGTTGT GGTCATGTAG CAAAAGAAAA TCGCCAAACA CAGGCTAATT 8008

TTGAGTGTGT AGAATGTGGC TACACAGAAA ATGCCGATGT GGTTGGAGCG TTAAATGTAT 8068

TGGGGCGTGG GCGAGCTATC GTCCACGCGT AATAAAATGT CAGGGCAGGA CATGCCCGTA 8128

GAGCTTGTGA AGTGAACTTC ATTGAGAGGT CAGCAACAAG AACCCACCGA GAGTAGCCCA 8188

TTGCTTGCCA ATTGGCACTA GTAGGAATCC CCATCCTTTA GGGCGGGGAG GATGTCAAAT 8248

ACATCATTAA TATTTAATGA AAAATATTAT AACTAATTGA TTTTTATATT ATTATTTGTC 8308

TATTTGGGCG GTGGGACATA ATTTTGACAG ACAGAATGAT ATCGTTTATA TTTCCGAATA 8368

TCTGAT ATG TTA TTT AGT AAA ATA TCA GAT AAG AAA AAT TTA TTT TTC 8416

Met Leu Phe Ser Lys Ile Ser Asp Lys Lys Asn Leu Phe Phe

1 5 10

TTT ATA TAT AGC TCA ATT AAA AGG AAA TTT ATT ATG AAA AAG ACA CTT 8464

Phe Ile Tyr Ser Ser Ile Lys Arg Lys Phe Ile Met Lys Lys Thr Leu

15 20 25 30

ATC GCT TTA GCT GTA ATA ACA ATG TTT TCA AGT GCA GCA AAT GCT GCG 8512

Ile Ala Leu Ala Val Ile Thr Met Phe Ser Ser Ala Ala Asn Ala Ala

35 40 45

GTC ATT TAT GAA AAA GAA GGT ACG AAA ATT GAT ATT GAT GGT CGT ATG 8560

Val Ile Tyr Glu Lys Glu Gly Thr Lys Ile Asp Ile Asp Gly Arg Met

50 55 60

CAT TTT GAA TTA CGT AAT GAT TCA GGC AAA CGT TCT GAT TTA CAA GAT 8608

His Phe Glu Leu Arg Asn Asp Ser Gly Lys Arg Ser Asp Leu Gln Asp

65 70 75

GCA GGC TCT CGT GTC CGC GTA AGA GCT TTT CAA GAA ATT GGC AAT GGC 8656

Ala Gly Ser Arg Val Arg Val Arg Ala Phe Gln Glu Ile Gly Asn Gly

80 85 90
 
TTT TCT ACC TAT GGG GCT GTT GAG TTT CGT TTT TCT ACT AAG AAA GAT 8704
Phe Ser Thr Tyr Gly Ala Val Glu Phe Arg Phe Ser Thr Lys Lys Asp
95 100 105 110
GGC TCA GAA CAA AGT ATT GGA TCT GAC TTA AGA GCT CAC CGC TTT TTT 8752
Gly Ser Glu Gln Ser Ile Gly Ser Asp Leu Arg Ala His Arg Phe Phe
115 120 125
GCA GGA ATT AAA CAA AAA GAC ATA GGG GAA TTA ACT TTC GGT AAA CAA 8800
Ala Gly Ile Lys Gln Lys Asp Ile Gly Glu Leu Thr Phe Gly Lys Gln
130 135 140
CTC CAT TTA GGT GAT CTT GTC CCG AAA GCA AAT TAT TCT TAT GAT TTA 8848
Leu His Leu Gly Asp Leu Val Pro Lys Ala Asn Tyr Ser Tyr Asp Leu
145 150 155
GGG GCG AAC TCT TTT TTT GGT GCA CAT AGT AAA GTA GCA CAT TTT ATT 8896
Gly Ala Asn Ser Phe Phe Gly Ala His Ser Lys Val Ala His Phe Ile
160 165 170
TCT GTA CCA TTT AAT GGT GTG AGG GTG TCT GCA G 8930
Ser Val Pro Phe Asn Gly Val Arg Val Ser Ala
175 180 185
SEQ ID NO.: 2
   SEQUENCE LENGTH: 13
   SEQUENCE TYPE: amino acid
   STRANDEDNESS: single
   TOPOLOGY: linear
   MOLECULE TYPE: peptide
   ORIGINAL SOURCE: Haemophilus paragallinarum serotype A strain 221
SEQUENCE DESCRIPTION: 
Lys Trp Leu Glu Val Tyr Ser Ser Ser Val Lys Leu Ser
1               5                   10
SEQ ID NO: 3
   SEQUENCE LENGTH: 43
   SEQUENCE TYPE: nucleic acid
   STRANDEDNESS: single
   TOPOLOGY: linear
   MOLECULE TYPE: other nucleic acid (synthetic DNA)
SEQUENCE DESCRIPTION: CGCGGATCCA TGAATAAAGT TTTTAAAATT AAATATTCTG TTG 43
SEQ ID NO.: 4
   SEQUENCE LENGTH: 39
   SEQUENCE TYPE: nucleic acid
   STRANDEDNESS: single
   TOPOLOGY: linear
   MOLECULE TYPE: other nucleic acid (synthetic DNA)
SEQUENCE DESCRIPTION: CGCGGATCCT TAAGGCTAAA AGCTAAATCC AACACTCAT 39
SEQ ID NO. : 5
   SEQUENCE LENGTH: 7486
   SEQUENCE TYPE: nucleic acid
   STRANDEDNESS: double
   TOPOLOGY: linear
   MOLECULE TYPE: genomic DNA
   ORIGINAL SOURCE: Haemophilus paragallinarum serotype C strain 53-47
SEQUENCE DESCRIPTION:

TCTAGAATAT AAATCTTCAG TATCAACATA CAAGGGGCGT ATCGCATACG CCCCTGTGCT 60

GAATTTTTAC TTACAGAACA CCGTACTTTT GTTCTGTCAT TTTTGGATAT TTGGGCTGAT 120

GGCTTTTTTC TTTGGTAGTT ATAACGGGTT TCGCTCGTTG AGCGACTTAC TTTCTTTTAC 180

ATTCCCAAAA GAAAGTAAGC AAAGAAAAGG GAACCCGACT AAATTGCTGT TGCTCATTCC 240

AATAAAATTT TCTTCATGAA AAGTAAGCCT GATGTTCGCT TCGCTCTCGC TCGGCGTTAC 300

TTTTCTTAAA ATTTTATTTC CATTCGGGCA ATTTACACGG GAAAGGGCGA TTTTTAAAAG 360

TGCGGTGGTT TTTGAAGGAT ATTTTTGTAT TTGGAAAAAT CAAGAAAATG GCTTTAGAAA 420

ACACCTCACT TATTTTAACT GTAGGTATTG CATTTTTAAT AATACAAATT TTTCTTGAAA 480

TGATGAAATA ACCAATCAAA TTAGTCAGTT ATAAGTGGAG AAACTTAAAG AAAATGATTA 540

AATTAGGCTC ACTCATTAGA CCAGTAAGGG AATTAAAATA GTATTTTTAA TTGCATTTAG 600

TTATTAAGTG TTAGAAATTA CCTATTGCAT CAATAAATGA GGTGTTTTTA TTTGTAATCT 660

CTAATTAATT AGAGTAGTAT TAAGTGGAGT TTTATCTTTA CTAAATTAAT GGTATCACCT 720

CTCAGAGAGG GAGAAGCAAA TTCCCCCCCC CTAGAAATAC CTAATAAGAG TTACATTAAG 780

GGCATATTAT AAAAGTAATT TATCAATAAT GATTAACGCT CATTATTATT AACAAAGGCA 840

AATGATT ATG AAT AAA GTT TTT AAA ATT AAA TAT TCT GTT GTA AAA CAA 889
Met Asn Lys Val Phe Lys Ile Lys Tyr Ser Val Val Lys Gln
-70 -65 -60

GAA ATG ATT GTG GTT TCA GAG CTA GCA AAT AAT AAA GAT AAA ACA GCT 937
Glu Met Ile Val Val Ser Glu Leu Ala Asn Asn Lys Asp Lys Thr Ala
-55 -50 -45

AGC CAA AAA AAC ACA CAT AAT ACT GCC TTT TTT CAA CCG CTA TTT ACA 985
Ser Gln Lys Asn Thr His Asn Thr Ala Phe Phe Gln Pro Leu Phe Thr
-40 -35 -30 -25

AAG TGT ACA TAT CTT GCT CTT CTC ATT AAT ATC GCA CTA GGA ACA TCA 1033
Lys Cys Thr Tyr Leu Ala Leu Leu Ile Asn Ile Ala Leu Gly Thr Ser
-20 -15 -10

TTA TTC CCT CAA TTA GCT AAT GCG AAA TTT TTA GAG GTT TAT AAT AGC 1081
Leu Phe Pro Gln Leu Ala Asn Ala Lys Phe Leu Glu Val Tyr Asn Ser
-5 1 5

TCC GTA AAA CTA CAG CAT GTT AAT AGT GGC GTA CCA AGT GAT AGT GTT 1129
Ser Val Lys Leu Gln His Val Asn Ser Gly Val Pro Ser Asp Ser Val
10 15 20

AAT CTT AAT CCA TCG GGA GGT GAG AAT GTT GGC ATG AAT AGC AAT CAA 1177
Asn Leu Asn Pro Ser Gly Gly Glu Asn Val Gly Met Asn Ser Asn Gln
25 30 35 40

GGG GTC GCT ATT GGC CGT GGT GCA GTA AAT AAT TAT TCG GCG ACG GGA 1225
Gly Val Ala Ile Gly Arg Gly Ala Val Asn Asn Tyr Ser Ala Thr Gly
45 50 55
TCA ATT GCT ATT GGT CAG GGG GCA AAA AAT GAT AAT TGG GCG ACG AGA 1273
Ser Ile Ala Ile Gly Gln Gly Ala Lys Asn Asp Asn Trp Ala Thr Arg
60 65 70
TCA ATT GCT ATT GGT CAG GGG GCA AAA AAT GAA AGT ATA GCA TCA GAT 1321
Ser Ile Ala Ile Gly Gln Gly Ala Lys Asn Glu Ser Ile Ala Ser Asp
75 80 85
TCT GTG GCT ATT TCC AAC GCG ATT AAC CGT TTT AAA AAA TCT ATT GTG 1369
Ser Val Ala Ile Ser Asn Ala Ile Asn Arg Phe Lys Lys Ser Ile Val
90 95 100
ATA GGT CTT AAT ACT TAT ACA CAA TTA GAT CCC CGT AGA GCT CCA GAA 1417
Ile Gly Leu Asn Thr Tyr Thr Gln Leu Asp Pro Arg Arg Ala Pro Glu
105 110 115 120
TCC CGT CAA GGT TCT GTG GTG ATT GGG GAA AAT GCG AAA AGT GCT GGG 1465
Ser Arg Gln Gly Ser Val Val Ile Gly Glu Asn Ala Lys Ser Ala Gly
125 130 135
AAT CAA TCT GTT TCT TTA GGG CAA AAT GCG TGG TCA AAA ACC AAT TCT 1513
Asn Gln Ser Val Ser Leu Gly Gln Asn Ala Trp Ser Lys Thr Asn Ser
140 145 150
ATT TCT ATT GGG GCA GGA ACC TTT GCG GAA GGG AAA TCA ACC ATT GCT 1561
Ile Ser Ile Gly Ala Gly Thr Phe Ala Glu Gly Lys Ser Thr Ile Ala
155 160 165
ATA GGG ACT GAT AAA ATA CTA GGG ACT AAT TAT AAT GAC AAA TTG CCT 1609
Ile Gly Thr Asp Lys Ile Leu Gly Thr Asn Tyr Asn Asp Lys Leu Pro
170 175 180
GCT CCT AGT TGG GAT GGA AGA ACA GGT AAG GCA CCT ACT AAT TCC ATT 1657
Ala Pro Ser Trp Asp Gly Arg Thr Gly Lys Ala Pro Thr Asn Ser Ile
185 190 195 200
TGG GAT ATA TTT TCT GAG TTA TAT ATG GGG AAA AAG ACT AAC GGC ACA 1705
Trp Asp Ile Phe Ser Glu Leu Tyr Met Gly Lys Lys Thr Asn Gly Thr
205 210 215

GAT TAT GAT GCA AAA AAA AAT GAC CGC GAT CCA AAT AAG [?]C[?]A [?]GAG G[?]T 1753
Asp Tyr Asp Ala Lys Lys Asn Asp Arg Asp Pro Asn Lys Pro Glu Ala
220 225 230
TTT TAT ACC TAT TCT GAT TTT AAA AGC AGA TAT GTT AAT AAC CCA AGT 1801
Phe Tyr Thr Tyr Ser Asp Phe Lys Ser Arg Tyr Val Asn Asn Pro Ser
235 240 245
ACC TCT CCC ACT TAT GCC GCT AAA TTA GGG GCA ATT GCC CTA GGT TCC 1849
Thr Ser Pro Thr Tyr Ala Ala Lys Leu Gly Ala Ile Ala Leu Gly Ser
250 255 260
CGC ACC ATT GCT GCG GGG GAA ATG TCC ACT GCG GTC GGT TCC TTA GCC 1897
Arg Thr Ile Ala Ala Gly Glu Met Ser Thr Ala Val Gly Ser Leu Ala
265 270 275 280
TTT GCA TTG GCA GAT AAA TCC ACC GCA ATG GGG TTA CGT TCT TTT GTT 1945
Phe Ala Leu Ala Asp Lys Ser Thr Ala Met Gly Leu Arg Ser Phe Val
285 290 295
GCT AAA GAT GCC GTA GGT GGA ACG GCA ATC GGG GAA GAA TCG CGA ACC 1993
Ala Lys Asp Ala Val Gly Gly Thr Ala Ile Gly Glu Glu Ser Arg Thr
300 305 310
TTT GCT AAA GAT TCC GTT GCC ATT GGT AAT AAA ACT GAA GCC TCA AAT 2041
Phe Ala Lys Asp Ser Val Ala Ile Gly Asn Lys Thr Glu Ala Ser Asn
315 320 325
GCT GGC TCA ATG GCT TAT GGT TAT AAG GCG AAA GCG GTA GGT GCG GGG 2089
Ala Gly Ser Met Ala Tyr Gly Tyr Lys Ala Lys Ala Val Gly Ala Gly
330 335 340
GCA ATC GCA ATT GGT GCA GAA GTC GCA GCA GGG GCT GAA TTT GAT AGC 2137
Ala Ile Ala Ile Gly Ala Glu Val Ala Ala Gly Ala Glu Phe Asp Ser
345 350 355 360
AGT CAA GCA GGA AAT TTA TTA CTA AAT AGA GGT GCT TAT GCT ACT TTA 2185
Ser Gln Ala Gly Asn Leu Leu Leu Asn Arg Gly Ala Tyr Ala Thr Leu
365 370 375
AAA AGT GCC GAT AAA TCA GAT GAT ATT AAA GCT GGA GAT GCG ATT AAC 2233
Lys Ser Ala Asp Lys Ser Asp Asp Ile Lys Ala Gly Asp Ala Ile Asn
380 385 390

GTA TTT ACC CAG TTT TTT GAT AAT ATG CTT ACT CAA GGC TCA CAC CTG 2281
Val Phe Thr Gln Phe Phe Asp Asn Met Leu Thr Gln Gly Ser His Leu
395 400 405
ACA TAT GAA AAT ACC ACC TAT TTA ACC ACT TCA GCA GGT GAT ATC AAG 2329
Thr Tyr Glu Asn Thr Thr Tyr Leu Thr Thr Ser Ala Gly Asp Ile Lys
410 415 420
AAA ACA TTA GCT GCA GTT GGA GAT GGC GGG AAA AAT GCC ATT GCC ATT 2377
Lys Thr Leu Ala Ala Val Gly Asp Gly Gly Lys Asn Ala Ile Ala Ile
425 430 435 440
GGT AAT AAA ACC TTT GCA TCT AAA GCA AAT TCT GTG GCA TTA GGG AGC 2425
Gly Asn Lys Thr Phe Ala Ser Lys Ala Asn Ser Val Ala Leu Gly Ser
445 450 455
TAT GCC TTA GCG AGT GCC CAA AAT GCC TTT GCA CTA GGT TCT TAT TCT 2473
Tyr Ala Leu Ala Ser Ala Gln Asn Ala Phe Ala Leu Gly Ser Tyr Ser
460 465 470
TTA GTG TCC CCT TTA GCA GCC AAT ACA ATC GTA ATT GGT GTG GGA GGT 2521
Leu Val Ser Pro Leu Ala Ala Asn Thr Ile Val Ile Gly Val Gly Gly
475 480 485
TAT GCC ACA GGA TCA AAC AGT TTC GTA GGG GGT TCT TGG GTA TCA ACC 2569
Tyr Ala Thr Gly Ser Asn Ser Phe Val Gly Gly Ser Trp Val Ser Thr
490 495 500
CTT TCA GCT CGG ACA GTT GTG CTA GGG TAT TCC GCT TCA ATT AGC TCA 2617
Leu Ser Ala Arg Thr Val Val Leu Gly Tyr Ser Ala Ser Ile Ser Ser
505 510 515 520
GAT TCT CAT GAT TCA TTA GCA ATG GGG GTG AAT GCC TTT ATT GGT AAT 2665
Asp Ser His Asp Ser Leu Ala Met Gly Val Asn Ala Phe Ile Gly Asn
525 530 535
GGT AGT AAT TCT TCA TTG GCA TTA GGT ACG GGA TCT ACT ATT GCG AAA 2713
Gly Ser Asn Ser Ser Leu Ala Leu Gly Thr Gly Ser Thr Ile Ala Lys
540 545 550
AAT GCC AAA TCT CCT GAC AGC TTA GCC ATT GGT AAA GAC TCA CGA ATT 2761
Asn Ala Lys Ser Pro Asp Ser Leu Ala Ile Gly Lys Asp Ser Arg Ile
555 560 565

GAC GCT AAA GAT ACA GAT AAT GGT GTT TTG TAT ACC CCT CAA GTT TAT 2809

Asp Ala Lys Asp Thr Asp Asn Gly Val Leu Tyr Thr Pro Gln Val Tyr

570 575 580

GAT GAA ACT ACT CGA GCC TTT AGA ACC TTT GAT GAA AAC AAA GAT TAT 2857

Asp Glu Thr Thr Arg Ala Phe Arg Thr Phe Asp Glu Asn Lys Asp Tyr

585 590 595 600

ATG CGT CAA GCA ATG GCA TTA GGT TTT AAT GCG AAG GTT TCG CGT GGG 2905

Met Arg Gln Ala Met Ala Leu Gly Phe Asn Ala Lys Val Ser Arg Gly

605 610 615

AAG GGC AAA ATG GAA ACG GGG ATT AAC TCG ATG GCG ATT GGT GCT CGT 2953

Lys Gly Lys Met Glu Thr Gly Ile Asn Ser Met Ala Ile Gly Ala Arg

620 625 630

TCT CAA GCA ACT TTG CAA AAT TCC ACC GCA CTT GGG GTA AAC GCT AAA 3001

Ser Gln Ala Thr Leu Gln Asn Ser Thr Ala Leu Gly Val Asn Ala Lys

635 640 645

ACA GAT TAC ACT TGG GAA CAG TTA GAA GCC GAT CCT TGG GTA TCT AAA 3049

Thr Asp Tyr Thr Trp Glu Gln Leu Glu Ala Asp Pro Trp Val Ser Lys

650 655 660

GGG GCA ATC AGT ATC CCA ACT TCA GGC AAA ATT GGG GTT ATC TCT GTG 3097

Gly Ala Ile Ser Ile Pro Thr Ser Gly Lys Ile Gly Val Ile Ser Val

665 670 675 680

GGC TCA AAA GGC TCA GAA CGT CGT ATT GTG AAT GTT GCT TCG GGT TCT 3145

Gly Ser Lys Gly Ser Glu Arg Arg Ile Val Asn Val Ala Ser Gly Ser

685 690 695

CTT GAT ACC GAT GCC GTG AAT GTT GCC CAA TTA AAA ACT ATT GAA GAA 3193

Leu Asp Thr Asp Ala Val Asn Val Ala Gln Leu Lys Thr Ile Glu Glu

700 705 710

CGT TTC CAA TCT GAA ATT GAT TTA TTA CAA AAT GGC GGT GGG GTG CAA 3241

Arg Phe Gln Ser Glu Ile Asp Leu Leu Gln Asn Gly Gly Gly Val Gln

715 720 725

TAT CTC TCT GTT GAA AAA ACG AAT ATC AAT GGA GAA GCG GGG AGA GTG 3289

Tyr Leu Ser Val Glu Lys Thr Asn Ile Asn Gly Glu Ala Gly Arg Val

730 735 740

GCT AGC CAA ATT CGT AAA GGG GAA AGT TAT AAG CGA TAT GTG AAA TTA 3337
Ala Ser Gln Ile Arg Lys Gly Glu Ser Tyr Lys Arg Tyr Val Lys Leu
745 750 755 760
AAA ACA CAA TTG CTC TAT TTA GAT GCA CGA AAA AAA TTA AAT GGA GAG 3385
Lys Thr Gln Leu Leu Tyr Leu Asp Ala Arg Lys Lys Leu Asn Gly Glu
765 770 775
AAG TTT GAT CAA ACT TCA TTA GAC AAA ATT AGT AAG GCA GTG CAA GAA 3433
Lys Phe Asp Gln Thr Ser Leu Asp Lys Ile Ser Lys Ala Val Gln Glu
780 785 790
CTT GAA GCG GAA TAT AGT GGC GAG TTA AAA ACA ACT GCG TCA GAA CTT 3481
Leu Glu Ala Glu Tyr Ser Gly Glu Leu Lys Thr Thr Ala Ser Glu Leu
795 800 805
AAT AGA GTT GCA ATG CAA TTG AAT GCT GAG ACA ACT GTA AAT GAC TTC 3529
Asn Arg Val Ala Met Gln Leu Asn Ala Glu Thr Thr Val Asn Asp Phe
810 815 820
GGG AAA TTT AAT CAA TAT AAA ACG CAG ATT GAG AAT GCA ACC AAT GCG 3577
Gly Lys Phe Asn Gln Tyr Lys Thr Gln Ile Glu Asn Ala Thr Asn Ala
825 830 835 840
GAT TCA GAA AAA AAT GTA GGC GGC TTA TCC CCT CAA GTA ATT GCA CAG 3625
Asp Ser Glu Lys Asn Val Gly Gly Leu Ser Pro Gln Val Ile Ala Gln
845 850 855
TTA AAA GCC AAT AAT AAC TAT CTT AAT GAT GGT GCA AAA GGG CAA GAC 3673
Leu Lys Ala Asn Asn Asn Tyr Leu Asn Asp Gly Ala Lys Gly Gln Asp
860 865 870
AGT ATA GCA TTT GGC TGG CAG GCA AAA ACC TCA GAA GCT AAT AAT GGA 3721
Ser Ile Ala Phe Gly Trp Gln Ala Lys Thr Ser Glu Ala Asn Asn Gly
875 880 885
TTA GCA GGG AAA CAA GCC ATT GCG ATT GGT TTC CAA GCG AAT TCT TCC 3769
Leu Ala Gly Lys Gln Ala Ile Ala Ile Gly Phe Gln Ala Asn Ser Ser
890 895 900
GCT GAA AAT GCC ATT TCT ATC GGT ACG AAT TCG GAT ACC TCA ATG ACA 3817
Ala Glu Asn Ala Ile Ser Ile Gly Thr Asn Ser Asp Thr Ser Met Thr
905 910 915 920

GGG GCA GTG GCG ATT GGT AAA GGT GCA ACG GTT ACT GCG GGT GGA AAA 3865
Gly Ala Val Ala Ile Gly Lys Gly Ala Thr Val Thr Ala Gly Gly Lys
925 930 935
CCT TCC ATT GCA TTG GGG CAA GAT TCG ACG GTT GCC AAT TCC GCA ATT 3913
Pro Ser Ile Ala Leu Gly Gln Asp Ser Thr Val Ala Asn Ser Ala Ile
940 945 950
AGC CGT ACA AGT TCA GTG ATG ATA AAT GGT TTA ACA TTC AAT AAT TTT 3961
Ser Arg Thr Ser Ser Val Met Ile Asn Gly Leu Thr Phe Asn Asn Phe
955 960 965
GCA GGT TCC CCT GAA ACA CTC GGT GTG TTA AGT ATC GGA ACG GCT GGG 4009
Ala Gly Ser Pro Glu Thr Leu Gly Val Leu Ser Ile Gly Thr Ala Gly
970 975 980
AAA GAG CGT AAA ATT GTT AAT GTT GCA GCA GGC GAT ATT TCG CAA ACT 4057
Lys Glu Arg Lys Ile Val Asn Val Ala Ala Gly Asp Ile Ser Gln Thr
985 990 995 1000
TCT ACT GAA GCC ATT AAC GGC TCA CAG CTT TAT GCA ACG AAC TTT ATG 4105
Ser Thr Glu Ala Ile Asn Gly Ser Gln Leu Tyr Ala Thr Asn Phe Met
1005 1010 1015
TTG AAC AAA CTG GCT CAA TCC GTT AAA ACG AAT TTT GGC GGT AAT GCA 4153
Leu Asn Lys Leu Ala Gln Ser Val Lys Thr Asn Phe Gly Gly Asn Ala
1020 1025 1030
AAC CTT GCC ACT GAT GGC ACA ATT ACA TTT ACA AAT ATT GGC GGC ACA 4201
Asn Leu Ala Thr Asp Gly Thr Ile Thr Phe Thr Asn Ile Gly Gly Thr
1035 1040 1045
GGG CAA GAT ACA ATC CAC GAT GCG ATT AAT AAT GTT CTC ACC AAA TTG 4249
Gly Gln Asp Thr Ile His Asp Ala Ile Asn Asn Val Leu Thr Lys Leu
1050 1055 1060
ATC TCG CTT TCG GCA ACA GAA GAA GAA GAA GTG GTG TCA GGG GAA GCT 4297
Ile Ser Leu Ser Ala Thr Glu Glu Glu Glu Val Val Ser Gly Glu Ala
1065 1070 1075 1080
GTC TAT GAT GCA CTT AAA GGT GCA AAA CCA ACG GTT TCA GCA GAA GCC 4345
Val Tyr Asp Ala Leu Lys Gly Ala Lys Pro Thr Val Ser Ala Glu Ala
1085 1090 1095

AAC AAA GGC ATT ACT GGC TTG GTG GAT GTG GTG AAA AAA GCA AAT TCA 4393
Asn Lys Gly Ile Thr Gly Leu Val Asp Val Val Lys Lys Ala Asn Ser
1100 1105 1110
CCG ATC ACA GTT GAG CCT TCT ACC GAT AAC AAC AAG AAA AAA ACC TTC 4441
Pro Ile Thr Val Glu Pro Ser Thr Asp Asn Asn Lys Lys Lys Thr Phe
1115 1120 1125
ACT GTC GGC TTA ATG AAA GAC ATT GAA GGG GTA AAC AGC ATT ACC TTT 4489
Thr Val Gly Leu Met Lys Asp Ile Glu Gly Val Asn Ser Ile Thr Phe
1130 1135 1140
GAT AAG TCA GGG CAA GAT CTA AAT CAA GTT ACG GGC AGA ATG AGC AGT 4537
Asp Lys Ser Gly Gln Asp Leu Asn Gln Val Thr Gly Arg Met Ser Ser
1145 1150 1155 1160
GCG GGT TTA ACC TTC AAA AAA GGC GAC ACA ACA AAT GGT TCA ACC ACC 4585
Ala Gly Leu Thr Phe Lys Lys Gly Asp Thr Thr Asn Gly Ser Thr Thr
1165 1170 1175
ACT TTT GCA GAA GAT GGC TTA ACC ATT GAT AGC ACA ACA AAT TCT GCT 4633
Thr Phe Ala Glu Asp Gly Leu Thr Ile Asp Ser Thr Thr Asn Ser Ala
1180 1185 1190
CAA ACA AAC TTA GTG AAA GTA AGT CGT GAT GGC TTC TCG GTG AAA AAT 4681
Gln Thr Asn Leu Val Lys Val Ser Arg Asp Gly Phe Ser Val Lys Asn
1195 1200 1205
GGC AGC GAT GAA AGC AAA TTA GCC TCG ACA AAA TTA TCT ATC GGT GCG 4729
Gly Ser Asp Glu Ser Lys Leu Ala Ser Thr Lys Leu Ser Ile Gly Ala
1210 1215 1220
GAA AAT GCA GAA CAC GTT GAA GTA ACT AAA TCG GGC ATA GCC TTA AAA 4777
Glu Asn Ala Glu His Val Glu Val Thr Lys Ser Gly Ile Ala Leu Lys
1225 1230 1235 1240
GCG GAT AAC ACC TCC GAT AAA TCT AGC ATC ACC TTA GCC CAA GAT GCG 4825
Ala Asp Asn Thr Ser Asp Lys Ser Ser Ile Thr Leu Ala Gln Asp Ala
1245 1250 1255
ATT ACT CTT GCG GGG AAC GCA ACC GGA ACG GCG ATT AAA TTG ACT GGT 4873
Ile Thr Leu Ala Gly Asn Ala Thr Gly Thr Ala Ile Lys Leu Thr Gly
1260 1265 1270

GTT GCA GAT GGC AAC ATT ACG GTA AAT TCA AAA GAT GCG GTA AAT GGG 4921
Val Ala Asp Gly Asn Ile Thr Val Asn Ser Lys Asp Ala Val Asn Gly
1275 1280 1285
GGG CAG TTG CGT ACC TTA TTA GGG GTT GAT AGC GGG GCT AAA ATT GGC 4969
Gly Gln Leu Arg Thr Leu Leu Gly Val Asp Ser Gly Ala Lys Ile Gly
1290 1295 1300
GGT ACT GAG AAA ACA ACG ATC AGT GAA GCC ATT TCT GAT GTG AAG CAA 5017
Gly Thr Glu Lys Thr Thr Ile Ser Glu Ala Ile Ser Asp Val Lys Gln
1305 1310 1315 1320
GCT CTT ACC GAT GCG ACA TTG GCA TAT AAA GCG GAC AAT AAA AAC GGT 5065
Ala Leu Thr Asp Ala Thr Leu Ala Tyr Lys Ala Asp Asn Lys Asn Gly
1325 1330 1335
AAA ACA GTT AAA TTG ACT GAC GGA TTG AAT TTT ACT AGC ACG ACC AAT 5113
Lys Thr Val Lys Leu Thr Asp Gly Leu Asn Phe Thr Ser Thr Thr Asn
1340 1345 1350
ATT GAT GCT TCA GTG GAA GAT AAC GGT GTG GTG AAA TTC ACC TTA AAA 5161
Ile Asp Ala Ser Val Glu Asp Asn Gly Val Val Lys Phe Thr Leu Lys
1355 1360 1365
GAT AAA TTA ACA GGC TTA AAA ACT ATC GCA ACT GAA TCT TTG AAT GCT 5209
Asp Lys Leu Thr Gly Leu Lys Thr Ile Ala Thr Glu Ser Leu Asn Ala
1370 1375 1380
TCT CAA AAT ATC ATC GCT GGC GGT ACG GTA ACA GTG GGC GGC GAG ACA 5257
Ser Gln Asn Ile Ile Ala Gly Gly Thr Val Thr Val Gly Gly Glu Thr
1385 1390 1395 1400
GAG GGC ATT GTG CTA ACA AAA TCT GGC TCA GGA AAT GAC CGC ACT TTA 5305
Glu Gly Ile Val Leu Thr Lys Ser Gly Ser Gly Asn Asp Arg Thr Leu
1405 1410 1415
TCT TTA TCT GGT GCA GGC AAT GCA GCA ACA GAT GGC ATT AAA GTC TCT 5353
Ser Leu Ser Gly Ala Gly Asn Ala Ala Thr Asp Gly Ile Lys Val Ser
1420 1425 1430
GGC GTG AAA GCA GGG ACG GCA GAC ACC GAT GCG GTG AAT AAA GGT CAG 5401
Gly Val Lys Ala Gly Thr Ala Asp Thr Asp Ala Val Asn Lys Gly Gln
1435 1440 1445

TTA GAT AAA CTT TTT AAA GCG ATC AAT GAC GCA TTA GGC ACA ACA GAT 5449
Leu Asp Lys Leu Phe Lys Ala Ile Asn Asp Ala Leu Gly Thr Thr Asp
1450 1455 1460
TTA GCG GTA ACC AAA AAT CCA AAT CAA ACC TCT ATC TTT AAT CCG ATA 5497
Leu Ala Val Thr Lys Asn Pro Asn Gln Thr Ser Ile Phe Asn Pro Ile
1465 1470 1475 1480
AAC GGC ACG GCT CCA ACC ACC TTT AAA GAC GCG GTG GAT AAA TTA ACC 5545
Asn Gly Thr Ala Pro Thr Thr Phe Lys Asp Ala Val Asp Lys Leu Thr
1485 1490 1495
ACC GCT GTG AAT ACA GGT TGG GGA TCA AAG GTA GGT ATT TTG GCA ACA 5593
Thr Ala Val Asn Thr Gly Trp Gly Ser Lys Val Gly Ile Leu Ala Thr
1500 1505 1510
GGT ATT GAT GGT ATT GAT GCT GGG AAT AAG AAA ATT AGT AAT GTC GCC 5641
Gly Ile Asp Gly Ile Asp Ala Gly Asn Lys Lys Ile Ser Asn Val Ala
1515 1520 1525
GAT GGG GAT ATT TCT CCA ACC AGT GGT GAT GTA GTG ACA GGT CGT CAG 5689
Asp Gly Asp Ile Ser Pro Thr Ser Gly Asp Val Val Thr Gly Arg Gln
1530 1535 1540
CTC TAC GCC TTA ATG CAG AAA GGT ATT CGC GTG TAT GGT GAT GAA GTT 5737
Leu Tyr Ala Leu Met Gln Lys Gly Ile Arg Val Tyr Gly Asp Glu Val
1545 1550 1555 1560
AGT CCA ACG AAG ACT CAA ACA ACA GCA CCT ACA GCA TCT AGC ACT CAA 5785
Ser Pro Thr Lys Thr Gln Thr Thr Ala Pro Thr Ala Ser Ser Thr Gln
1565 1570 1575
GGT GGG GCG ACA ACG GCG AAT ACG GCG GGT GGT GTA GCA CCA GCA GGT 5833
Gly Gly Ala Thr Thr Ala Asn Thr Ala Gly Gly Val Ala Pro Ala Gly
1580 1585 1590
AAT GTA GCA ACG GGG GAT ATT GCG CCG ACA CAG CCA GCA TTG CCA GAG 5881
Asn Val Ala Thr Gly Asp Ile Ala Pro Thr Gln Pro Ala Leu Pro Glu
1595 1600 1605
ATG AAA ACG GCA TTG GTT GGT GAT CAC TTG GCT GTG CCG TTA GGT GGA 5929
Met Lys Thr Ala Leu Val Gly Asp His Leu Ala Val Pro Leu Gly Gly
1610 1615 1620

AGC CTC AAG ATT CAC GGA GAT CAT AAT GTG AAA ACA ACG ATT TCT CCG 5977
Ser Leu Lys Ile His Gly Asp His Asn Val Lys Thr Thr Ile Ser Ala
1625 1630 1635 1640
GGT AAT CAA GTG GGG ATT TCA TTA CAG CCA AAT ATT TCT ATT GAG AAT 6025
Gly Asn Gln Val Gly Ile Ser Leu Gln Pro Asn Ile Ser Ile Glu Asn
1645 1650 1655
AAC TTG GTA ATT GGT TCA AAT AAG CCT GAG AAG GCA AAA TTA GCC GCA 6073
Asn Leu Val Ile Gly Ser Asn Lys Pro Glu Lys Ala Lys Leu Ala Ala
1660 1665 1670
CAA GAA GGT AAT GCT TTG GTT ATC ACT AAC AAA GAT GAC GGG AAT GCG 6121
Gln Glu Gly Asn Ala Leu Val Ile Thr Asn Lys Asp Asp Gly Asn Ala
1675 1680 1685
GCG ATG GTC TTT AAT AAC GAG AAA AAT ATG CTT GTT CTC AGT GAT AAA 6169
Ala Met Val Phe Asn Asn Glu Lys Asn Met Leu Val Leu Ser Asp Lys
1690 1695 1700
AAG GCA AAA CCA AGA GCG GTT CTT GAT GGA CAA AAT GGG GCA TTA ACT 6217
Lys Ala Lys Pro Arg Ala Val Leu Asp Gly Gln Asn Gly Ala Leu Thr
1705 1710 1715 1720
TTA GTC GGC AAT GAT GAT TCT CAA GTC ACC CTT TCC TCT AAG AAA GGT 6265
Leu Val Gly Asn Asp Asp Ser Gln Val Thr Leu Ser Ser Lys Lys Gly
1725 1730 1735
AAA GAT ATT GAT GGA AAT GAT TTG AGC CGT CTC TCT GTG ACG ACT GAA 6313
Lys Asp Ile Asp Gly Asn Asp Leu Ser Arg Leu Ser Val Thr Thr Glu
1740 1745 1750
AGA ACA AAT GCT GAT GGG CAA CTT GAA AAA GTG GAA ACC TCA TTT GCT 6361
Arg Thr Asn Ala Asp Gly Gln Leu Glu Lys Val Glu Thr Ser Phe Ala
1755 1760 1765
ACA ATG GAT GAT GGC TTG AAG TTC AAA GCC GAC GGG GAT AAA GTG ATT 6409
Thr Met Asp Asp Gly Leu Lys Phe Lys Ala Asp Gly Asp Lys Val Ile
1770 1775 1780
AAT AAG AAA CTT AAT GAA ACC GTT GAA ATT GTT GGT GAT GAG AAT GTG 6457
Asn Lys Lys Leu Asn Glu Thr Val Glu Ile Val Gly Asp Glu Asn Val
1785 1790 1795 1800

ACA ACA TCT ATT ACT GAT GAT AAT AAG GTG AAA GTT TCA CTG AAT AAG 6509
Thr Thr Ser Ile Thr Asp Asp Asn Lys Val Lys Val Ser Leu Asn Lys
1805 1810 1815
AAA ATC GCG ATT GAT GAG GTT AAG ATT CCA AAT ACA GAT CCT GAT GCT 6553
Lys Ile Ala Ile Asp Glu Val Lys Ile Pro Asn Thr Asp Pro Asp Ala
1820 1825 1830
CAA AAG GGA GAT AGC ATT GTA ATC AAC AAT GGT GGA ATC CAC GCA GGT 6601
Gln Lys Gly Asp Ser Ile Val Ile Asn Asn Gly Gly Ile His Ala Gly
1835 1840 1845
AAT AAA GTG ATT ACT GGC GTT AAA GCG AGT GAT GAC CCA ACC AGT GCG 6649
Asn Lys Val Ile Thr Gly Val Lys Ala Ser Asp Asp Pro Thr Ser Ala
1850 1855 1860
GTG AAT CGA GGT CAA TTA AAT ACT GTG ATT GAT AAT GTT CAA AAT AAT 6697
Val Asn Arg Gly Gln Leu Asn Thr Val Ile Asp Asn Val Gln Asn Asn
1865 1870 1875 1880
TTC AAT CAA GTT AAT CAA CGT ATT GGC GAT TTA ACA CGG GAG TCG CGT 6745
Phe Asn Gln Val Asn Gln Arg Ile Gly Asp Leu Thr Arg Glu Ser Arg
1885 1890 1895
GCA GGT ATT GCA GGT GCA ATG GCG ACG GCA AGC CTA CAA AAT GTT GCT 6793
Ala Gly Ile Ala Gly Ala Met Ala Thr Ala Ser Leu Gln Asn Val Ala
1900 1905 1910
TTA CCA GGG AAA ACA ACG ATT TCC GTA GGT ACA GCA ACG TTC AAA GGG 6841
Leu Pro Gly Lys Thr Thr Ile Ser Val Gly Thr Ala Thr Phe Lys Gly
1915 1920 1925
GAG AAT GCT GTT GCA ATA GGG ATG TCT AGA CTC TCT GAT AAT GGA AAA 6889
Glu Asn Ala Val Ala Ile Gly Met Ser Arg Leu Ser Asp Asn Gly Lys
1930 1935 1940
GTA GGT ATC CGT TTA TCT GGT ATG AGT ACA AGT AAC GGA GAT AAA GGG 6937
Val Gly Ile Arg Leu Ser Gly Met Ser Thr Ser Asn Gly Asp Lys Gly
1945 1950 1955 1960
GCA GCA ATG AGT GTT GGA TTT ACC TTT TAGCCTTAAT CCATAAATAA GCAAAA[?] 6991
Ala Ala Met Ser Val Gly Phe Thr Phe
1965

GCGAATCACC TTTGATTCGC TTTTTTTATC AGATTATGTG CCGTAAAACT CCGTCCTTCA 7051

GGGCGGAGAT ATAAGGCACA AACGGCGTAA GCCGTTTCAA ACCTAACTAA TCAGGTGTTT 7111

GTTGTTGCTC AATGTATTGG CGAATAATGG AAATTGGAGT GCCACCACAA CTCCCTGCAA 7171

AATAAGACGG AGACCAAAGC TGATTACCCC AAAGTTTTTT GCGGATGTTC GAGTAGTTTT 7231

TCTTCCTAAT CATTCGGCTT GATACACCTT TTAAACTGTT TACAAGTGTA GATACAGCCA 7291

CTTTCGGTGG ATATTCCACA AGTAAATGAA CATGATCGTC TTCACCGTCA AATTCAACTA 7351

ATTTTGCTTT AAAATCATTG CAGACGCTTT CAAAAATCAA TTTGAGTTCG TCTAAAATAG 7411

CTTTCGTAAA AACATCACAG CGATATTTTG TTACAAAGAC TAAGTGAACA TGCATATTAA 7471

AAACACAATG TCTAC 7486

## Claims

1. A recombinant polypeptide from Haeamophilus paragallinarum capable of inducing production of HI antibody and/or protecting against avian infectious coryza selected from the group consisting of
(a) polypeptides having the amino acid sequence as shown in SEQ ID NO: 1 or in SEQ ID NO:5;
(b) polypeptides having the amino acid sequence at the N-terminal end as shown in SEQ NO: 2 and having a molecular weight of about 130 kD;
(c) polypeptides having the amino acid sequence encoded by the nucleotide sequence of from nucleotide residues NO: 2212 to NO: 6275 of SEQ ID NO: 1; and
(d) polypeptides having the amino acid sequence encoded by the nucleotide residues NO:1 to NO: 3450 of SECT ID NO: 1.

2. The polypeptide of claim 1 wherein Haemophilus paragallinarum is Haemophilus paragallinarum serotype A or serotype C.

3. The polypeptide of claim 1 or 2, wherein one or several amino acid residues are deleted, added or substituted.

4. A DNA having a nucleotide sequence coding for the polypeptide of any one of claims 1 to 3.

5. The DNA of claim 4 which comprises the nucleotide sequence of SEQ ID NO: 1 or SEQ ID NO: 5.

6. A DNA coding for a polypeptide from Haemophilus paragallinarum capable of inducing production of HI antibody and/or protecting against avian infectious coryza which hybridizes with a DNA of a nucleotide sequence complementary to the nucleotide sequence as shown in SEQ ID NO: 1 or SEPA ID NO: 5.

7. A recombinant DNA molecule comprising the DNA of any one of claims 4 to 6.

8. The recombinant DNA molecule of claim 7 wherein a vector of said recombinant DNA molecule is selected from the group consisting of a plasmid, a viral vector and a cosmid.

9. A transformant cell transformed with either the DNA of any one of claims 4 to 6 or the recombinant DNA molecule as set forth in claim 7 or 8.

10. The transformant cell of claim 9 which is a host selected from the group consisting of bacteria, yeast, insect cell, animal cell and plant cell.

11. An antibody which recognizes the polypeptide of any one of claims 1 to 3.

12. The antibody of claim 11 which is a monoclonal antibody or a polyclonal antibody.

13. A process for preparing the polypeptide as set forth in claim 1 or 3 by using monoclonal antibody having HI activity.

14. The process of claim 13 wherein said polypeptide is derived from Haemophilus paragallinarum.

15. The process of claim 14 wherein Haemophilus paragallinarum is Haemophilus paragallinarum serotype A or serotype C.

16. A process for preparing the polypeptide of any one of claims 1 to 3 which comprises culturing the transformant cell as set forth in claim 9 or 10 so that said transformant cell could produce the polypeptide of any one of claims 1 to 3, and purifying said polypeptide:

17. An immunogenic composition comprising the polypeptide of any one of claims 1 to 3, the DNA of any one of claims 4 to 6, the recombinant DNA molecule as set forth in claim 7 or 8 or the transformant cell as set forth in claim 9 or 10 and, optionally, a suitable carrier, diluent or stabilizing agent.

18. The immunogenic composition according to claim 17 for protection against avian infectious coryza.

19. A therapeutic agent comprising as an active ingredient the antibody as set forth in claim 11 or 12, the polypeptide of any one of claims 1 to 3 or the vector of claim 8 and, optionally, a suitable carrier, diluent or stabilizing agent.

20. A therapeutic agent according to claim 19 for avian infectious coryza.

21. A vaccine composition comprising a recombinant polypeptide from Haemophilus paragallinarum capable of inducing production of HI antibody and/or protecting against avian infectious coryza selected from the group consisting of:
(a) polypeptides having the amino acid sequence at the N-terminal end as shown in SEQ NO: 2 and having a molecular weight of about 130 kD;
(b) polypeptides having the amino acid sequence encoded by the nucleotide sequence of from nucleotide residues NO: 2212 to NO: 6275 of SEQ ID NO: 1; and
(c) polypeptides having the amino acid sequence encoded by the nucleotide residues NO:1 to NO: 3450 of SEQ ID NO: 1.

22. The vaccine according to claim 21 for protection against avian infectious coryza

## Patentansprüche

1. Rekombinantes Polypeptid aus Haemophilus paragallinarum, das fähig ist, die Produktion von HI-Antikörper auszulösen und/oder gegen Vogel-infektiöse Coryza zu schützen, ausgewählt aus der Gruppe bestehend aus:
(a) Polypeptiden, die eine Aminosäuresequenz haben, wie in SEQ ID NO:1 oder in SEQ ID NO:5 gezeigt;
(b) Polypeptiden, die eine Aminosäuresequenz am N-terminalen Ende haben, wie in SEQ ID NO:2 gezeigt, und ein Molekulargewicht von etwa 130 kD;
(c) Polypeptiden, die eine Aminosäuresequenz haben, die codiert wird durch die Nucleotidsequenz von den Nucleotidresten NO:2212 bis NO:6275 der SEQ ID NO:1; und
(d) Polypeptiden, die eine Aminosäuresequenz haben, die codiert wird durch die Nucleotidreste NO:1 bis NO:3450 der SEQ m NO: 1.

2. Polypeptid nach Anspruch 1, wobei Haemophilus paragallinarum ein Haemophilus paragallinarum-Serotyp A oder -Serotyp C ist.

3. Polypeptid nach Anspruch 1 oder 2, wobei eine oder mehrere Aminosäurereste entfernt, hinzugefügt oder ersetzt sind.

4. DNA, die eine Nucleotidsequenz hat, die ein Polypeptid nach einem der Ansprüche 1 bis 3 codiert.

5. DNA nach Anspruch 4, welche die Nucleotidsequenz von SEQ ID NO:1 oder SEQ ID NO:5 umfasst.

6. DNA, die ein Polypeptid aus Haemophilus paragallinarum codiert, das fähig ist, die Produktion von HI-Antikörpern auszulösen und/oder gegen Vogel-infektiöse Coryza zu schützen, welche mit einer DNA einer Nucleotidsequenz hybridisiert, die komplementär ist zur Nucleotidsequenz, die in SEQ ID NO:1 1 oder SEQ ID NO: 5 gezeigt ist.

7. Rekombinantes DNA-Molekül, umfassend die DNA nach einem der Ansprüche 4 bis 6.

8. Rekombinantes DNA-Molekül nach Anspruch 7, wobei ein Vektor dieses rekombinanten DNA-Moleküls ausgewählt ist aus der Gruppe bestehend aus einem Plasmid, einem viralen Vektor und einem Cosmid.

9. Transformierte Zelle, die transformiert ist mit entweder der DNA nach einem der Ansprüche 4 bis 6 oder dem rekombinanten DNA-Molekül wie in Ansprüchen 7 oder 8 ausgeführt.

10. Transformierte Zelle nach Anspruch 9, die ein Wirt ist ausgewählt aus der Gruppe bestehend aus Bakterien, Hefe, Insektenzelle, Tierzelle und Pflanzenzelle.

11. Antikörper, der das Polypeptid nach einem der Ansprüche 1 bis 3 erkennt.

12. Antikörper nach Anspruch 11, der ein monoclonaler oder polyclonaler Antikörper ist.

13. Verfahren zur Herstellung des Polypeptids, wie in Anspruch 1 oder 3 ausgeführt, durch Verwendung eines monoclonalen Antikörpers, der HI-Aktivität hat.

14. Verfahren nach Anspruch 13, wobei das Polypeptid von Haemophilus paragallinarum stammt.

15. Verfahren nach Anspruch 14, wobei Haemophilus paragallinarum ein Haemophilus paragallinarum-Serotyp A oder -Serotyp C ist.

16. Verfahren zur Herstellung des Polypeptids nach einem der Ansprüche 1 bis 3, welches das Züchten der transformierten Zelle, wie in Anspruch 9 oder 10 ausgeführt, umfasst, so dass diese transformierte Zelle das Polypeptid nach einem der Ansprüche 1 bis 3 herstellen kann, und das Reinigen dieses Polypeptids.

17. Immunogene Zusammensetzung, umfassend das Polypeptid nach einem der Ansprüche 1 bis 3, die DNA nach einem Ansprüche 4 bis 6, das rekombinante DNA-Molekül wie in Anspruch 7 oder 8 ausgeführt oder die transformierte Zelle wie in Anspruch 9 oder 10 ausgeführt, und gegebenenfalls einen geeigneten Träger, ein Diluens oder ein stabilisierendes Agens.

18. Immunogene Zusammensetzung nach Anspruch 17 zum Schutz gegen Vogel-infektiöse Coryza.

19. Therapeutisches Agens, umfassend als aktiven Bestandteil den Antikörper wie in Anspruch 11 oder 12 beschrieben, das Polypeptid nach einem der Ansprüche 1 bis 3 oder den Vektor nach Anspruch 8 und gegebenenfalls einen geeigneten Träger, ein Diluens oder ein stabilisierendes Agens.

20. Therapeutisches Agens nach Anspruch 19 gegen Vogel-infektiöse Coryza.

21. Impfstoff-Zusammensetzung umfassend ein rekombinantes Polypeptid aus Haemophilus paragallinarum, das fähig ist, die Produktion von HI-Antikörpern auszulösen und/oder gegen Vogel-infektiöse Coryza zu schützen, ausgewählt aus der Gruppe bestehend aus:
(a) Polypeptiden, die die Aminosäuresequenz am N-terminalen Ende wie in SEQ ID NO:2 gezeigt haben und ein Molekulargewicht von etwa 130 kD;
(b) Polypeptiden, die die Aminosäuresequenz haben, die codiert wird durch die Nucleotidsequenz von Nucleotidresten NO:2212 bis NO:6275 der SEQ ID NO:1; und
(c) Polypeptiden, die die Aminosäuresequenz haben, die codiert wird durch die Nucleotidreste NO:1 1 bis NO:3450 der SEQ ID NO:1.

22. Impfstoff nach Anspruch 21 zum Schutz gegen Vogel-infektiöse Coryza.

## Revendications

1. Polypeptide recombinant provenant d'Haeamophilus paragallinarum capable d'induire la production d'anticorps HI et/ou de protéger contre le coryza infectieux aviaire choisi dans le groupe constitué des :
(a) polypeptides ayant la séquence d'acides aminés telle que montrée dans SEQ ID NO : 1 ou dans SEQ ID NO :5 ;
(b) polypeptides ayant la séquence d'acides aminés située à l'extrémité N-terminale telle que montrée dans SEQ ID NO:2 et ayant un poids moléculaire d'environ 130 kD ;
(c) polypeptides ayant la séquence d'acides aminés codée par la séquence nucléotidique allant des résidus nucléotidiques NO : 2212 à NO : 6275 de SEQ ID NO : 1 ; et
(d) polypeptides ayant la séquence d'acides aminés codée par les résidus nucléotidiques NO :1 à NO : 3450 de SEQ ID NO : 1.

2. Polypeptide selon la revendication 1 dans lequel Haeamophilus paragallinarum est l'Haeamophilus paragallinarum de sérotype A ou de sérotype C.

3. Polypeptide selon la revendication 1 ou 2, dans lequel un ou plusieurs résidus d'acides aminés sont supprimés, ajoutés ou substitués.

4. ADN ayant une séquence nucléotidique codant pour le polypeptide selon l'une quelconque des revendications 1 à 3.

5. ADN selon la revendication 4 qui comprend la séquence nucléotidique selon SEQ ID NO : 1 ou SEQ ID NO : 5.

6. ADN codant pour un polypeptide provenant d'Haeamophilus paragallinarum capable d'induire la production d'anticorps HI et/ou de protéger contre le coryza infectieux aviaire qui s'hybride avec un ADN dont la séquence nucléotidique est complémentaire de la séquence nucléotidique telle que montrée dans SEQ ID NO : 1 ou SEQ ID NO : 5.

7. Molécule d'ADN recombinant comprenant l'ADN de l'une quelconque des revendications 4 à 6.

8. Molécule d'ADN recombinant selon la revendication 7 dans laquelle un vecteur de ladite molécule d'ADN recombinant est choisi dans le groupe constitué d'un plasmide, d'un vecteur viral et d'un cosmide.

9. Cellule transformante transformée à l'aide soit d'un ADN selon l'une quelconque des revendications 4 à 6 soit de la molécule d'ADN recombinant telle que définie dans la revendication 7 ou 8.

10. Cellule transformante selon la revendication 9 qui est un hôte sélectionné à partir du groupe constitué de bactérie, levure, cellule d'insecte, cellule animale et cellule végétale.

11. Anticorps qui reconnaît le polypeptide selon l'une quelconque des revendications 1 à 3.

12. Anticorps selon la revendication 11 qui est un anticorps monoclonal ou un anticorps polyclonal.

13. Procédé pour préparer le polypeptide tel que défini dans la revendication 1 ou 3 en utilisant un anticorps monoclonal ayant une activité HI.

14. Procédé selon la revendication 13 dans lequel ledit polypeptide est dérivé d'Haemophilus paragallinarum.

15. Procédé selon la revendication 14 dans lequel Haeamophilus paragallinarum est l'Haeamophilus paragallinarum de sérotype A ou de sérotype C.

16. Procédé pour préparer le polypeptide selon l'une quelconque des revendications 1 à 3 qui comprend la culture de la cellule transformante telle que définie dans la revendication 9 ou 10 de sorte que ladite cellule transformante puisse produire le polypeptide selon l'une quelconque des revendications 1 à 3, et la purification dudit polypeptide.

17. Composition immunogène comprenant le polypeptide selon l'une quelconque des revendications 1 à 3, l'ADN selon l'une quelconque des revendications 4 à 6, la molécule d'ADN recombinant telle que définie dans la revendication 7 ou 8 ou la cellule transformante telle que définie dans la revendication 9 ou 10 et, éventuellement, un support, un diluant ou un agent stabilisant adapté(s).

18. Composition immunogène selon la revendication 17 en vue d'assurer une protection contre le coryza infectieux aviaire.

19. Agent thérapeutique comprenant en tant qu'ingrédient actif l'anticorps tel que défini dans la revendication 11 ou 12, le polypeptide selon l'une quelconque des revendications 1 à 3 ou le vecteur selon la revendication 8 et, éventuellement, un support, un diluant ou un agent stabilisant adapté(s).

20. Agent thérapeutique selon la revendication 19 pour le coryza infectieux aviaire.

21. Composition vaccinale comprenant un polypeptide recombinant provenant d'Haemophilus paragallinarum capable d'induire la production d'anticorps HI et/ou de protéger contre le coryza infectieux aviaire choisi dans le groupe constitué des :
(a) polypeptides ayant la séquence d'acides aminés située à l'extrémité N-terminale telle que montrée dans SEQ ID NO :2 et ayant un poids moléculaire d'environ 130 kD ;
(b) polypeptides ayant la séquence d'acides aminés codée par la séquence nucléotidique allant des résidus nucléotidiques NO : 2212 à NO : 6275 de SEQ ID NO : 1 ; et
(c) polypeptides ayant la séquence d'acides aminés codée par les résidus nucléotidiques NO :1 à NO : 3450 de SEQ ID NO : 1.

22. Vaccin selon la revendication 21 en vue d'assurer une protection contre le coryza infectieux aviaire.
